# EUROPEAN PATENT SPECIFICATION

(11) **EP 1 963 259 B1**
(45) Date of publication and mention of the grant of the patent: **15.02.2012**
(21) Application number: 06820474.2
(22) Date of filing: 12.12.2006
(51) Int. Cl.: C07D 257/04, C07C 317/22, C07C 323/65, C07C 311/51, A61K 31/18, A61P 11/00, A61K 31/275

(54) **SUBSTITUTED DIPHENYLETHERS, -AMINES, -SULFIDES AND -METHANES FOR THE TREATMENT OF RESPIRATORY DISEASE**
SUBSTITUIERTE DIPHENYLETHER, -AMINE, -SULFIDE UND METHANE ZUR BEHANDLUNG VON ATEMWEGSERKRANKUNGEN
DIPHENYL-ETHERS, -AMINES, -SULFIDES ET -METHANES SUBSTITUES POUR LE TRAITEMENT DE MALADIES RESPIRATOIRES

(30) Priority: 15.12.2005 GB 0525477; 13.04.2006 GB 0607409; 26.07.2006 GB 0614787
(43) Date of publication of application: 03.09.2008
(60) Divisional application: 10173760.9
(73) Proprietor: AstraZeneca AB, 151 85 Södertälje (SE)
(72) Inventor: BONNERT, Roger, Victor, Leicestershire LE11 5RH (GB); LUKER, Timothy, Jon, Leicestershire LE11 5RH (GB); MOHAMMED, Rukhsana, Tasneem, Leicestershire LE11 5RH (GB); THOM, Stephen, Leicestershire LE11 5RH (GB); COOK, Andrew, Leicestershire LE11 5RH (GB)
(86) International application number: PCT/GB2006/004607
(87) International publication number: WO 2007/068894

(56) References cited:
- WO-A-2004/089885
- WO-A-2005/018529
- WO-A-2005/044260
- WO-A-2005/105727

## Description

The present invention relates to substituted aromatic compounds as useful pharmaceutical compounds for treating respiratory disorders, pharmaceutical compositions containing them, and processes for their preparation.

Compounds that are active at the CRTH2 receptor are known in the art as potentially useful for the treatment of various respiratory diseases, including asthma and COPD.

WO2005018529 contains related compounds which are phenoxyacetic acids. However, they are very acidic and potentially prone to formation of acyl glucuronide metabolites. The present invention discloses novel acid bioisosteres replacing the carboxylic acid group which have potentially different physical properties.

In a first aspect the invention therefore provides compound of formula (I) or a pharmaceutically acceptable salt thereof: 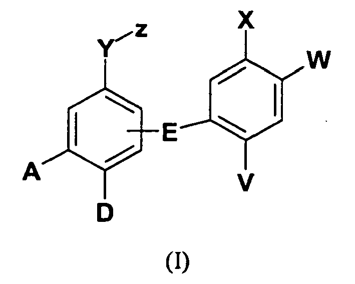 in which:
A and D are independently selected from hydrogen, halogen, phenyl or C₁₋₃ alkyl, the latter being optionally substituted by halogen atoms;
E is O, S, NR^{3'} or CH₂;
V is halogen, CN or C₁₋₃alkyl, the latter being optionally substituted by halogen atoms;
W is selected from SO₂R⁶ and COR⁴;
X is hydrogen or CF₃;
Y is selected from O-CR¹R², CR¹R² and CR¹R²CR¹R²;
Z is CONHSO₂R⁶;
R¹ and R² independently represent a hydrogen atom, halogen, C₃₋₈ cycloalkyl or a C₁-₆ alkyl group, the latter two groups being optionally substituted by one or more halogen atoms; or
R¹ and R² together can form a 3-8 membered ring optionally containing one or more atoms selected from O, S, NR³ and itself optionally substituted by one or more halogen, C₃₋₈ cycloalkyl or C₁₋₃ alkyl the latter two groups being optionally substituted by one or more halogen or NR⁴R⁵;
R³ represents hydrogen, C₃₋₈ cycloalkyl or C₁₋₆ alkyl the latter two groups being optionally substituted by halogen atoms;
R^{3'} represents hydrogen, C₃₋₈ cycloalkyl or C₁₋₆ alkyl the latter two groups being optionally substituted by halogen atoms;
R⁴ and R⁵ independently represent hydrogen, C₃₋₈ cycloalkyl or C₁₋₆alkyl the latter two groups being optionally substituted by one or more substituents independently selected from halogen, CN, C₃₋₇ cycloalkyl, C₁₋₆ alkyl, OR³ and NR⁷R⁸, aryl, heteoaryl, S(O)ₙR⁹ (where n = 0,1 or 2), CONR⁷R⁸, NR³COR¹⁰, SO₂NR⁴R⁵ and NR³SO₂R⁹;
   or
R⁴ and R⁵ together with the nitrogen atom to which they are attached can form a 3-8 membered saturated ring optionally containing one or more atoms selected from O, N, S(O)ₙ (where n = 0,1 or 2), NR³, and itself optionally substituted by one or more halogen, OR³, C₃₋₈ cycloalkyl or C₁₋₆ alkyl, the latter two groups being optionally substituted by one or more halogen;
R⁶ represents aryl, heteroaryl, C₃₋₈ cycloalkyl or C₁₋₆alkyl all of which being optionally substituted by one or more substituents independently selected from halogen, C₃₋₇ cycloalkyl, C₁₋₆ alkyl, OR³, CN, NR⁷R⁸, aryl, heteoaryl, S(O)ₙR⁹ (where n = 0,1 or 2), CONR⁷R⁸, NR₃COR¹⁰, SO₂NR⁴R⁵ and NR³SO₂R⁹;
R⁷ and R⁸ independently represent hydrogen, C₃₋₈ cycloalkyl or C₁₋₆alkyl, the latter two groups being optionally substituted by one or more halogen atoms;
   or
R⁷ and R⁸ together with the nitrogen atom to which they are attached can form a 3-8 membered saturated ring, optionally substituted by halogen, C₃₋₈ cycloalkyl or C₁₋₃ alkyl;
R⁹ represents C₃₋₈ cycloalkyl or C₁₋₆ alkyl (optionally substituted by halogen atoms);
R¹⁰ represents hydrogen, aryl, heteroaryl, OR₃, C₃₋₈ cycloalkyl or C₁-₆ alkyl the latter three groups being optionally substituted by halogen atoms;
R¹¹ and R¹² independently represent hydrogen, aryl, heteroaryl, C₃₋₈ cycloalkyl or C₁₋₈ alkyl, the latter being optionally substituted by one or more halogen atoms, OR³, =O or NR¹³R¹⁴;
   or
R¹¹ and R¹² together with the nitrogen atom to which they are attached can form a 3-8 membered ring optionally containing one or more atoms selected from O, S, NR³. This ring is itself optionally substituted by one or more C₃₋₈ cycloalkyl, C₁₋₃ alkyl, halogen, OR³ or NR¹³R¹⁴.
R¹³ and R¹⁴ independently represent hydrogen, C₃₋₈ cycloalkyl or C₁₋₆ alkyl the latter two groups being optionally substituted by one or more substituents independently selected from halogen, OR³, CN, S(O)ₙR⁹ (where n = 0,1 or 2), CONR⁷R⁸, NR³COR¹⁰, SO₂NR⁴R⁵ and NR³SO₂R⁹;
   or
R¹³ and R¹⁴ together with the nitrogen atom to which they are attached can form a 5-10 membered unsaturated heteroaryl ring optionally containing one or more atoms selected from O, N, S(O)ₙ(where n = 0,1 or 2), NR³, and itself optionally substituted by halogen, CN, S(O)ₙR⁹ (where n = 0, 1 or 2), OR³, C₃₋₈ cycloalkyl or C₁₋₃ alkyl.
R¹⁵ represents one or more substituents independently selected from hydrogen, halogen, CN, OR³, S(O)ₙR⁹ (where n = 0, 1 or 2), C₃₋₈ cycloalkyl or C₁₋₆ alkyl the latter two groups being optionally substituted by halogen atoms,
provided that:
• A and D cannot both be hydrogen

In the context of the present specification, unless otherwise indicated, an alkyl or alkenyl group or an alkyl or alkenyl moiety in a substituent group may be linear, branched or cyclic and may be optionally substituted by one or more halogen atoms.

Examples of aryl include phenyl, biphenyl and naphthyl.

Heteroaryl is defined as a 5-7 member aromatic ring or can be 6,6- or 6,5-fused bicyclic ring optionally containing one or more heteroatoms selected from N, S, O. The bicyclic ring may be linked through carbon or nitrogen and may be attached through the 5 or 6 membered ring and can be fully or partially saturated.

Examples include but are not limited to pyridine, pyrimidine, thiazole, oxazole, pyrazole, imidazole, furan, isoxazole, pyrrole, isothiazole and azulene, naphthyl, indene, quinoline, isoquinoline, indole, indolizine, benzo[b]furan, benzo[b]thiophene, 1H-indazole, benzimidazole, benzthiazole, benzoxazole, purine, 4H-quinolizine, cinnoline, phthalazine, quinazoline, quinoxaline, 1,8-naphthyridine, pteridine, quinolone and 1,2-methylenedioxy benzene.

Aryl or heteroaryl groups as substituents can be optionally substituted by one or more substituents independently selected from halogen, CN, OR⁷, SO₂R³, CONR⁷R⁸, SO₂NR⁴R⁵, C₃₋₈ cycloalkyl or C₁₋₆ alkyl, the latter two groups being optionally substituted by one or more substituents independently selected from halogen, OR³.

Examples of heterocyclic aromatic rings as for G include but are not limited to oxazoles, imidazoles, thiazoles, isoxazoles, pyrazoles, isothiazoles, oxadiazoles, thiadiazoles, triazoles, tetrazoles, oxatriazoles and thiatriazoles.

Tautomers and isomers form an aspect of the invention, in particular where R¹⁵ is equal to hydroxy, then G will include all possible tautomeric forms thereof.

Preferably A and D are independently selected from hydrogen, halogen or C₁₋₃ alkyl, the latter being optionally substituted by halogen atoms; most preferably, A and D are independently selected from hydrogen, halogen or CF₃.

Preferably A is hydrogen when D is halogen or C₁₋₃ alkyl, the latter being optionally substituted by halogen atoms; when D is hydrogen A is preferably halogen, phenyl or C₁₋₃ alkyl, the latter being optionally substituted by halogen atoms;

Preferably E is O or S.

Preferably Y is CR¹R².

Preferably W is SO₂R⁶.

Most preferably W is SO₂methyl, SO₂ethyl, SO₂aryl, SO₂CH₂aryl or SO₂CH₂heteroaryl.

Preferably R¹ and R² are both hydrogen.

Preferably E is connected to the aromatic ring in the meta-position with respect to Y.

Preferably when Y is a bond, then A is C₁-₃ alkyl, the latter being optionally substituted by halogen atoms; preferably D is hydrogen.

Preferably when Y is a bond, then E is O.

Preferably when Y is a bond, then V is halogen.

Preferably when Y is a bond, then R⁶ is C₁₋₆alkyl.

Preferred compounds of the invention include:
2-[2-[2-chloro-4-(methylsulfonyl)phenoxy]-4-(trifluoromethyl)phenoxy]-*N-*(methylsulfonyl)acetamide
2-{4-chloro-3-[2-chloro-4-(methylsulfonyl)phenoxy]phenyl}-*N*-(methylsulfonyl) acetamide
2-{3-chloro-5-[2-chloro-4-(methylsulfonyl)phenoxy]phenyl}-*N*-(methylsulfonyl) acetamide
2-(3-chloro-5-{[2-fluoro-4-(phenylsulfonyl)phenyl]thio}phenyl)-N-(methylsulfonyl) acetamide
2-[3-{2-chloro-4-[(4-fluorobenzyl)sulfonyl]phenoxy}-5-(trifluoromethyl)phenyl]-*N-*(methylsulfonyl)acetamide
2-[3-[2-chloro-4-(methylsulfonyl)phenoxy]-5-(trifluoromethyl)phenyl]-*N-*(methylsulfonyl)acetamide
2-[3-[2-cyano-4-(phenylsulfonyl)phenoxy]-5-(trifluoromethyl)phenyl]-*N-*(methylsulfonyl)acetamide
2-[3-[4-(Ethylsulfonyl)-2-(trifluoromethyl)phenoxy]-5-(trifluoromethyl)phenyl]-*N-*(methylsulfonyl)acetamide
2-[3-[2-cyano-4-(phenylsulfonyl)phenoxy]-5-(trifluoromethyl)phenyl]-*N-*(ethylsulfonyl)acetamide
2-[3-[2-chloro-4-(phenylsulfonyl)phenoxy]-5-(trifluoromethyl)phenyl]-N-(methylsulfonyl)acetamide
2-[3-[2-chloro-4-(phenylsulfonyl)phenoxy]-5-(trifluoromethyl)phenyl]-N-(ethylsulfonyl)acetamide
2-[3-[2-chloro-4-(ethylsulfonyl)phenoxy]-5-(trifluoromethyl)phenyl]-N-(ethylsulfonyl)propanamide
2-[3-{[2-chloro-4-(ethylsulfonyl)phenyl]thio}-5-(trifluoromethyl)phenyl]-N-(ethylsulfonyl)acetamide
2-[3-{[2-chloro-4-(ethylsulfonyl)phenyl]thio}-5-(trifluoromethyl)phenyl]-N-(methylsulfonyl)acetamide
N-(tert-butylsulfonyl)-2-[3-[2-cyano-4-(phenylsulfonyl)phenoxy]-5-(trifluoromethyl) phenyl]acetamide
2-[3-[2-cyano-4-(phenylsulfonyl)phenoxy]-5-(trifluoromethyl)phenyl]-N-(isopropyl sulfonyl)acetamide
2-[3-[2-chloro-4-(methylsulfonyl)phenoxy]-5-(trifluoromethyl)phenyl]-N-[(2,2,2-trifluoroethyl)sulfonyl]acetamide
2-(4-chloro-2-{[2-chloro-4-(methylsulfonyl)phenyl]amino}phenoxy)-N-(methylsulfonyl) acetamide
2-[3-[2-chloro-4-(methylsulfonyl)phenoxy]-5-(trifluoromethyl)phenyl]-N-(ethylsulfonyl) acetamide
2-[3-[2-chloro-4-(methylsulfonyl)phenoxy]-5-(trifluoromethyl)phenyl]-N-(propylsulfonyl)acetamide
N-(benzylsulfonyl)-2-[3-[2-chloro-4-(methylsulfonyl)phenoxy]-5-(trifluoromethyl)phenyl]acetamide
N-(ethylsulfonyl)-2-[3-[4-(methylsulfonyl)-2-(trifluoromethyl)phenoxy]-5-(trifluoromethyl)phenyl]acetamide
2-[3-[4-(methylsulfonyl)-2-(trifluoromethyl)phenoxy]-5-(trifluoromethyl)phenyl]-N-(propylsulfonyl)acetamide
2-{5-[2-chloro-4-(ethylsulfonyl)phenoxy]biphenyl-3-yl}-N-(ethylsulfonyl)acetamide
2-{3-chloro-5-[4-(methylsulfonyl)-2-(trifluoromethyl)phenoxy]phenyl}-N-[(trifluoromethyl)sulfonyl]acetamide
2-{3-chloro-5-[2-chloro-4-(4-fluorobenzoyl)phenoxy]phenyl}-N-[(2,2,2-trifluoroethyl)sulfonyl]acetamide
2-[3-[2-cyano-4-(phenylsulfonyl)phenoxy]-5-(trifluoromethyl)phenyl]-N-(propylsulfonyl)acetamide
2-{3-[2-chloro-4-(ethylsulfonyl)phenoxy]-5-fluorophenyl}-N-(propylsulfonyl)acetamide
2-{3-chloro-5-[2-chloro-4-(ethylsulfonyl)phenoxy]phenyl}-N-(propylsulfonyl)acetamide
2-(3-chloro-5-[2-chloro-4-(ethylsulfonyl)phenoxy]phenyl}-N-(ethylsulfonyl)acetamide
2-{3-chloro-5-[2-chloro-4-(ethylsulfonyl)phenoxy]phenyl}-*N*-(isobutylsulfonyl)acetamide and pharmaceutically acceptable salts thereof.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

The compound of formula (I) above may be converted to a pharmaceutically acceptable salt or solvate thereof, preferably a basic addition salt such as sodium, potassium, calcium, aluminium, lithium, magnesium, zinc, benzathine, chloroprocaine, choline, diethanolamine, ethanolamine, ethyldiamine, tertiarybutylamine, meglumine, tromethamine or procaine, or an acid addition salt such as a hydrochloride, hydrobromide, phosphate, acetate, fumarate, maleate, tartrate, citrate, oxalate, methanesulphonate or *p-*toluenesulphonate.

It will be appreciated by those skilled in the art that in the processes of the present invention certain functional groups in the starting reagents or intermediate compound may need to be protected by protecting groups. Thus, the preparation of the compound of formula (I) may involve, at an appropriate stage, the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

Compounds of formula (I) in which Z is C(O)NHSO₂R⁶ can be synthesised as outlined in Scheme (1) in which compounds of formula (XX) are converted to the acid chloride using a reagent such as oxalyl chloride and subsequently reacted with a sulfonamide of formula (XXI) using a suitable base such as hunigs base in a suitable solvent such as DCM. Alternatively compounds of formula (XX) can be directly coupled with sulfonamides of formula (XXI) using a suitable coupling agent such as PyBOP or HATU or CDI with a suitable base such as Hunigs base or DBU in a suitable solvent such as DCM or THF.

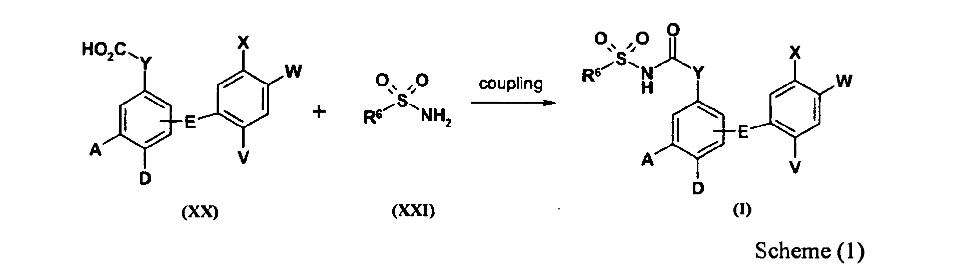

Compounds of formula (XX) or protected derivatives thereof where Y is OCH₂ can be prepared using literature procedures by those skilled in the art, or by methods described in WO2005018529.

Compounds of formula (XX) or protected derivatives thereof where Y is CH₂ can be prepared as described below in Scheme (2)

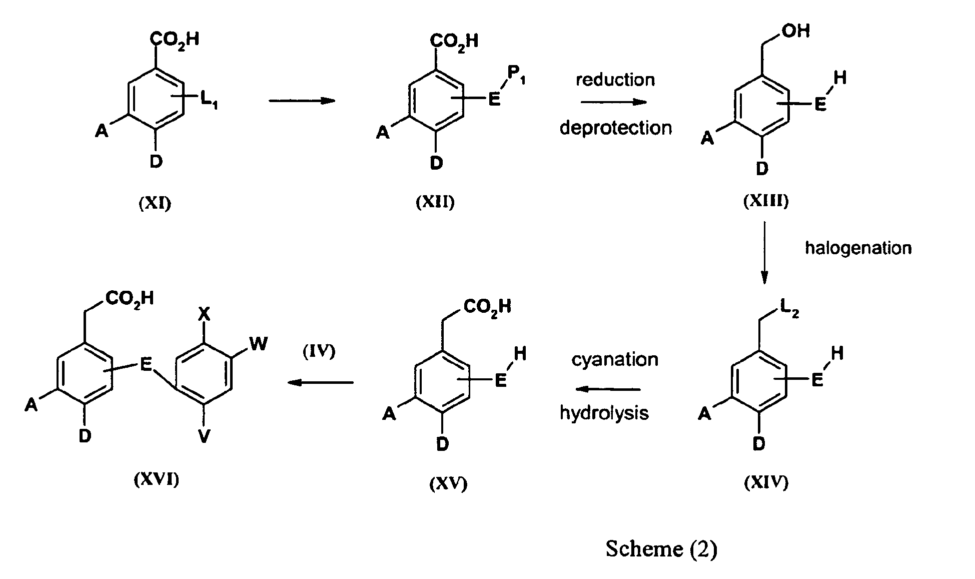

In which A, D, E, V, W and X are as described for compounds of formula (I) or are protected derivatives thereof. L¹ is a suitable leaving group such as fluorine or chlorine. L² is a halogen atom such as chlorine or bromine. P¹ is a suitable protecting group preferably alkyl. The group L¹ contained in compounds of formula (XI) is displaced using for example sodium methoxide in a polar solvent such as DMPU or HMPA at elevated temperatures to give compounds of formula (XII). When the group P¹ is alkyl it is deprotected using boron tribromide. The acid is converted to the benzyl alcohol using a suitable reducing agent, preferably borane or lithium aluminium hydride in a suitable organic solvent such as THF. The alcohol is then halogenated using either thionyl chloride or thionyl bromide in a solvent such as DCM containing DMF. The halogen is then displaced with sodium or potassium cyanide at elevated temperatures in a solvent such as DMSO and the nitrile hydrolysed with a suitable reagent such as HBr to give compounds of formula (XV). Compounds of formula (XV) are coupled with compounds of formula (IV) as described in Scheme (1), to afford compounds of formula (XVI).

Compounds of formula (XXI) or protected derivatives thereof are commercially available or can be prepared readily using literature procedures by those skilled in the art.

Compounds of formula (I) in which Y is CR¹R² and Z is C(O)NHSO₂R⁶ can be synthesised according to Scheme (3):-

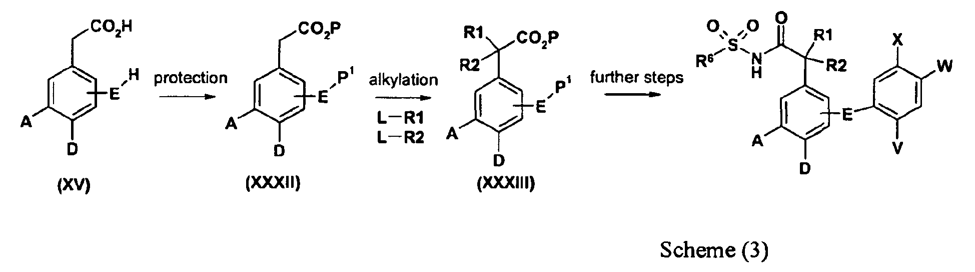

In which A, D, E, V, W, X, R¹ and R² are as described for compounds of formula (I) or are protected derivatives thereof. P is a suitable protecting group such as methyl, ethyl or t-butyl. L is a suitable leaving group such as halogen. Compounds of formula (XV) are protected using standard conditions such as reaction with acidic methanol or treatment with iodomethane in the presence of a suitable base, such as potassium carbonate, in a suitable solvent, such as DMF, to give compounds of formula (XXXII). Alkylation is carried out using standard conditions such as deprotonation with a suitable base such as t-BuLi, n-BuLi or NaH in a suitable solvent such as THF followed by addition of the alkylating agent at suitable temperatures usually between -78°C and 0°C to give compounds of formula (XXXIII). Subsequent deprotection, coupling with compounds of formula (IV) and sulphonamide formation are carried out using standard methods as described above.

Compounds of formula (I) in which A is CN, C₁₋₆ alkyl, aryl or heteroaryl and in which Z is C(O)NHSO₂R⁶ can be prepared from compounds of formula (XX) as outlined in Scheme (4):

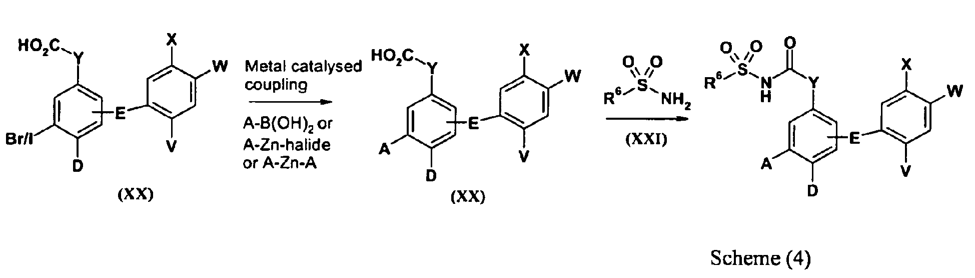

In which A, D, E, V, W, X, Y and R⁶ are as defined in formula (I) or are protected derivatives thereof. The reaction is carried out using standard metal-catalysed coupling techniques. For example, the coupling reactions can be carried out by reacting compounds of formula (XX) with an appropriate activated palladium catalyst such as bisdiphenylphosphino ferrocene palladium (II) and with the boronic acid adduct of A in the presence of a suitable base such as sodium carbonate or potassium carbonate or cesium carbonate in a suitable solvent such as toluene, THF or dioxane. The reactions are usually carried out at elevated temperatures, for example 80°C. Alternatively, the coupling reactions can be carried out by reacting compounds of formula (XIVa) with an appropriate activated palladium catalyst such as bisdiphenylphosphinoferrocene palladium (II) and with the zinc adduct of A at elevated temperatures, for example 80°C, in a suitable solvent such as toluene, THF or dioxane. Compounds of formula (I) are obtained by coupling with sulfonamides of formula (XXI) as described above.

The compounds of formula (I) or pharmaceutically acceptable salts thereof have activity as pharmaceuticals, in particular as modulators of CRTh2 receptor activity, and may be used in the treatment (therapeutic or prophylactic) of conditions/diseases in human and non-human animals which are exacerbated or caused by excessive or unregulated production of PGD₂ and its metabolites.

A compound of the invention, or a pharmaceutically acceptable salt thereof, can be used in the treatment of:
1. respiratory tract: obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
2. bone and joints: arthritides associated with or including osteoarthritis/osteoarthrosis, both primary and secondary to, for example, congenital hip dysplasia; cervical and lumbar spondylitis, and low back and neck pain; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease;
   primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;
3. pain and connective tissue remodelling of musculoskeletal disorders due to injury [for example sports injury] or disease: arthitides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);
4. skin: psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;
5. eyes: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral , fungal, and bacterial;
6. gastrointestinal tract: glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);
7. abdominal: hepatitis, including autoimmune, alcoholic and viral; fibrosis and cirrhosis of the liver; cholecystitis; pancreatitis, both acute and chronic;
8. genitourinary: nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
9. allograft rejection: acute and chronic following, for example, transplantation of kidney, heart, liver, lung, bone marrow, skin or cornea or following blood transfusion; or chronic graft versus host disease;
10. CNS: Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;
11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;
12. other disorders with an inflammatory or immunological component; including acquired immune deficiency syndrome (AIDS), leprosy, Sezary syndrome, and paraneoplastic syndromes;
13. cardiovascular: atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;
14. oncology: treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; and,
15. gastrointestinal tract: Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema.
16. Diseases associated with raised levels of PGD₂ or its metabolites.

Thus, the present invention provides a compound of formula (I), or a pharmaceutically-acceptable salt or solvate thereof, as hereinbefore defined for use in therapy.

Preferably the compounds of the invention are used to treat diseases in which the chemokine receptor belongs to the CRTh2 receptor subfamily.

Particular conditions which can be treated with the compounds of the invention are asthma, rhinitis and other diseases in which raised levels of PGD₂ or its metabolites. It is preferred that the compounds of the invention are used to treat asthma.

In a further aspect, the present invention provides the use of a compound of formula (I), or a pharmaceutically acceptable salt or solvate thereof, as hereinbefore defined in the manufacture of a medicament for use in therapy.

In a further aspect, the present invention provides the use of a compound or formula (I), or a pharmaceutically acceptable salt or solvate thereof, as hereinbefore defined in the manufacture of a medicament for use in therapy in combination with drugs used to treat asthma and rhinitis (such as inhaled and oral steroids, inhaled β2-receptor agonists and oral leukotriene receptor antagonists).

The invention further relates to combination therapies wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for the treatment of one or more of the conditions listed.

In particular, for the treatment of the inflammatory diseases such as (but not restricted to) rheumatoid arthritis, osteoarthritis, asthma, allergic rhinitis, chronic obstructive pulmonary disease (COPD), psoriasis, and inflammatory bowel disease, the compounds of the invention may be combined with agents listed below.

Non-steroidal anti-inflammatory agents (hereinafter NSAIDs) including non-selective cyclo-oxygenase COX-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate; leflunomide; hydroxychloroquine; d-penicillamine; auranofin or other parenteral or oral gold preparations; analgesics; diacerein; intra-articular therapies such as hyaluronic acid derivatives; and nutritional supplements such as glucosamine.

The present invention still further relates to the combination of a compound of the invention, or a pharmaceutically acceptable salt thereof, together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-α) inhibitors such as anti-TNF monoclonal antibodies (for example infliximab; adalimumab, and CDP-870) and TNF receptor antagonists including immunoglobulin molecules (such as etanercept) and low-molecular-weight agents such as pentoxyfylline.

In addition the invention relates to a combination of a compound of the invention, or a pharmaceutically acceptable salt thereof, with a monoclonal antibody targeting B-Lymphocytes (such as CD20 (rituximab), MRA-aIL16R and T-Lymphocytes, CTLA4-Ig, HuMax Il-15).

The present invention still further relates to the combination of a compound of the invention, or a pharmaceutically acceptable salt thereof, with a modulator of chemokine receptor function such as an antagonist of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family.

The present invention further relates to the combination of a compound of the invention, or a pharmaceutically acceptable salt thereof, with an inhibitor of matrix metalloprotease (MMPs), i.e., the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-9 and MMP-12, including agents such as doxycycline.

The present invention still further relates to the combination of a compound of the invention, or a pharmaceutically acceptable salt thereof, and a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; a N-(5-substituted)-thiophene-2-alkylsulfonamide; 2,6-di-tert-butylphenolhydrazones; a methoxytetrahydropyrans such as Zeneca ZD-2138; the compound SB-210661; a pyridinyl-substituted 2-cyanonaphthalene compound such as L-739,010; a 2-cyanoquinoline compound such as L-746,530; or an indole or quinoline compound such as MK-591, MK-886, and BAY x 1005.

The present invention further relates to the combination of a compound of the invention, or a pharmaceutically acceptable salt thereof, and a receptor antagonist for leukotrienes (LT) B4, LTC4, LTD4, and LTE4. selected from the group consisting of the phenothiazin-3-1s such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention, or a pharmaceutically acceptable salt thereof, and a phosphodiesterase (PDE) inhibitor such as a methylxanthanine including theophylline and aminophylline; a selective PDE isoenzyme inhibitor including a PDE4 inhibitor an inhibitor of the isoform PDE4D, or an inhibitor of PDE5.

The present invention further relates to the combination of a compound of the invention, or a pharmaceutically acceptable salt thereof, and a histamine type 1 receptor antagonist such as cetirizine, loratadine, desloratadine, fexofenadine, acrivastine, terfenadine, astemizole, azelastine, levocabastine, chlorpheniramine, promethazine, cyclizine, or mizolastine; applied orally, topically or parenterally.

The present invention still further relates to the combination of a compound of the invention, or a pharmaceutically acceptable salt thereof, and a proton pump inhibitor (such as omeprazole) or a gastroprotective histamine type 2 receptor antagonist.

The present invention further relates to the combination of a compound of the invention, or a pharmaceutically acceptable salt thereof, and an antagonist of the histamine type 4 receptor.

The present invention still further relates to the combination of a compound of the invention, or a pharmaceutically acceptable salt thereof, and an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride or ethylnorepinephrine hydrochloride.

The present invention further relates to the combination of a compound of the invention, or a pharmaceutically acceptable salt thereof, and an anticholinergic agents including muscarinic receptor (M1, M2, and M3) antagonist such as atropine, hyoscine, glycopyrrrolate, ipratropium bromide, tiotropium bromide, oxitropium bromide, pirenzepine or telenzepine.

The present invention still further relates to the combination of a compound of the invention, or a pharmaceutically acceptable salt thereof, and a beta-adrenoceptor agonist (including beta receptor subtypes 1-4) such as isoprenaline, salbutamol, formoterol, salmeterol, terbutaline, orciprenaline, bitolterol mesylate, or pirbuterol, or a chiral enantiomer thereof.

The present invention further relates to the combination of a compound of the invention, or a pharmaceutically acceptable salt thereof, and a chromone, such as sodium cromoglycate or nedocromil sodium.

The present invention still further relates to the combination of a compound of the invention, or a pharmaceutically acceptable salt thereof, with a glucocorticoid, such as flunisolide, triamcinolone acetonide, beclomethasone dipropionate, budesonide, fluticasone propionate, ciclesonide or mometasone furoate.

The present invention further relates to the combination of a compound of the invention, or a pharmaceutically acceptable salt thereof, with an agent that modulates a nuclear hormone receptor such as PPARs.

The present invention still further relates to the combination of a compound of the invention, or a pharmaceutically acceptable salt thereof, together with an immunoglobulin (Ig) or Ig preparation or an antagonist or antibody modulating Ig function such as anti-IgE (for example omalizumab).

The present invention further relates to the combination of a compound of the invention, or a pharmaceutically acceptable salt thereof, and another systemic or topically-applied anti-inflammatory agent, such as thalidomide or a derivative thereof, a retinoid, dithranol or calcipotriol.

The present invention still further relates to the combination of a compound of the invention, or a pharmaceutically acceptable salt thereof, and combinations of aminosalicylates and sulfapyridine such as sulfasalazine, mesalazine, balsalazide, and olsalazine; and immunomodulatory agents such as the thiopurines, and corticosteroids such as budesonide.

The present invention further relates to the combination of a compound of the invention, or a pharmaceutically acceptable salt thereof, together with an antibacterial agent such as a penicillin derivative, a tetracycline, a macrolide, a beta-lactam, a fluoroquinolone, metronidazole, an inhaled aminoglycoside; an antiviral agent including acyclovir, famciclovir, valaciclovir, ganciclovir, cidofovir, amantadine, rimantadine, ribavirin, zanamavir and oseltamavir; a protease inhibitor such as indinavir, nelfinavir, ritonavir, and saquinavir; a nucleoside reverse transcriptase inhibitor such as didanosine, lamivudine, stavudine, zalcitabine or zidovudine; or a non-nucleoside reverse transcriptase inhibitor such as nevirapine or efavirenz.

The present invention still further relates to the combination of a compound of the invention, or a pharmaceutically acceptable salt thereof, and a cardiovascular agent such as a calcium channel blocker, a beta-adrenoceptor blocker, an angiotensin-converting enzyme (ACE) inhibitor, an angiotensin-2 receptor antagonist; a lipid lowering agent such as a statin or a fibrate; a modulator of blood cell morphology such as pentoxyfylline; thrombolytic, or an anticoagulant such as a platelet aggregation inhibitor.

The present invention further relates to the combination of a compound of the invention, or a pharmaceutically acceptable salt thereof, and a CNS agent such as an antidepressant (such as sertraline), an anti-Parkinsonian drug (such as deprenyl, L-dopa, ropinirole, pramipexole, a MAOB inhibitor such as selegine and rasagiline, a comP inhibitor such as tasmar, an A-2 inhibitor, a dopamine reuptake inhibitor, an NMDA antagonist, a nicotine agonist, a dopamine agonist or an inhibitor of neuronal nitric oxide synthase), or an anti-Alzheimer's drug such as donepezil, rivastigmine, tacrine, a COX-2 inhibitor, propentofylline or metrifonate.

The present invention still further relates to the combination of a compound of the invention, or a pharmaceutically acceptable salt thereof, and an agent for the treatment of acute or chronic pain, such as a centrally or peripherally-acting analgesic (for example an opioid or derivative thereof), carbamazepine, phenytoin, sodium valproate, amitryptiline or other anti-depressant agent-s, paracetamol, or a non-steroidal anti-inflammatory agent.

The present invention further relates to the combination of a compound of the invention, or a pharmaceutically acceptable salt thereof, together with a parenterally or topically-applied (including inhaled) local anaesthetic agent such as lignocaine or a derivative thereof.

A compound of the present invention, or a pharmaceutically acceptable salt thereof, can also be used in combination with an anti-osteoporosis agent including a hormonal agent such as raloxifene, or a biphosphonate such as alendronate.

The present invention still further relates to the combination of a compound of the invention, or a pharmaceutically acceptable salt thereof, together with a: (i) tryptase inhibitor; (ii) platelet activating factor (PAF) antagonist; (iii) interleukin converting enzyme (ICE) inhibitor; (iv) IMPDH inhibitor; (v) adhesion molecule inhibitors including VLA-4 antagonist; (vi) cathepsin; (vii) kinase inhibitor such as an inhibitor of tyrosine kinase (such as Btk, Itk, Jak3 or MAP, for example Gefitinib or Imatinib mesylate), a serine / threonine kinase (such as an inhibitor of a MAP kinase such as p38, JNK, protein kinase A, B or C, or IKK), or a kinase involved in cell cycle regulation (such as a cylin dependent kinase); (viii) glucose-6 phosphate dehydrogenase inhibitor; (ix) kinin-B.sub1. - or B.sub2. -receptor antagonist; (x) anti-gout agent, for example colchicine; (xi) xanthine oxidase inhibitor, for example allopurinol; (xii) uricosuric agent, for example probenecid, sulfinpyrazone or benzbromarone; (xiii) growth hormone secretagogue; (xiv) transforming growth factor (TGFβ); (xv) platelet-derived growth factor (PDGF); (xvi) fibroblast growth factor for example basic fibroblast growth factor (bFGF); (xvii) granulocyte macrophage colony stimulating factor (GM-CSF); (xviii) capsaicin cream; (xix) tachykinin NK.sub1. or NK.sub3. receptor antagonist such as NKP-608C, SB-233412 (talnetant) or D-4418; (xx) elastase inhibitor such as UT-77 or ZD-0892; (xxi) TNF-alpha converting enzyme inhibitor (TACE); (xxii) induced nitric oxide synthase (iNOS) inhibitor; (xxiii) chemoattractant receptor-homologous molecule expressed on TH2 cells, (such as a CRTH2 antagonist); (xxiv) inhibitor of P38; (xxv) agent modulating the function of Toll-like receptors (TLR), (xxvi) agent modulating the activity of purinergic receptors such as P2X7; or (xxvii) inhibitor of transcription factor activation such as NFkB, API, or STATS.

A compound of the invention, or a pharmaceutically acceptable salt thereof, can also be used in combination with an existing therapeutic agent for the treatment of cancer, for example suitable agents include:
(i) an antiproliferative/antineoplastic drug or a combination thereof, as used in medical oncology, such as an alkylating agent (for example cis-platin, carboplatin, cyclophosphamide, nitrogen mustard, melphalan, chlorambucil, busulphan or a nitrosourea); an antimetabolite (for example an antifolate such as a fluoropyrimidine like 5-fluorouracil or tegafur, raltitrexed, methotrexate, cytosine arabinoside, hydroxyurea, gemcitabine or paclitaxel); an antitumour antibiotic (for example an anthracycline such as adriamycin, bleomycin, doxorubicin, daunomycin, epirubicin, idarubicin, mitomycin-C, dactinomycin or mithramycin); an antimitotic agent (for example a vinca alkaloid such as vincristine, vinblastine, vindesine or vinorelbine, or a taxoid such as taxol or taxotere); or a topoisomerase inhibitor (for example an epipodophyllotoxin such as etoposide, teniposide, amsacrine, topotecan or a camptothecin);
(ii) a cytostatic agent such as an antioestrogen (for example tamoxifen, toremifene, raloxifene, droloxifene or iodoxyfene), an oestrogen receptor down regulator (for example fulvestrant), an antiandrogen (for example bicalutamide, flutamide, nilutamide or cyproterone acetate), a LHRH antagonist or LHRH agonist (for example goserelin, leuprorelin or buserelin), a progestogen (for example megestrol acetate), an aromatase inhibitor (for example as anastrozole, letrozole, vorazole or exemestane) or an inhibitor of 5α-reductase such as finasteride;
(iii) an agent which inhibits cancer cell invasion (for example a metalloproteinase inhibitor like marimastat or an inhibitor of urokinase plasminogen activator receptor function);
(iv) an inhibitor of growth factor function, for example: a growth factor antibody (for example the anti-erbb2 antibody trastuzumab, or the anti-erbb1 antibody cetuximab [C225]), a farnesyl transferase inhibitor, a tyrosine kinase inhibitor or a serine/threonine kinase inhibitor, an inhibitor of the epidermal growth factor family (for example an EGFR family tyrosine kinase inhibitor such as N-(3-chloro-4-fluorophenyl)-7-methoxy-6-(3-morpholinopropoxy)quinazolin-4-amine (gefitinib), N-(3-ethynylphenyl)-6,7-bis(2-methoxyethoxy)quinazolin-4-amine (erlotinib, OSI-774) or 6-acrylamido-N-(3-chloro-4-fluorophenyl)-7-(3-morpholinopropoxy)quinazolin-4-amine (CI 1033)), an inhibitor of the platelet-derived growth factor family, or an inhibitor of the hepatocyte growth factor family;
(v) an antiangiogenic agent such as one which inhibits the effects of vascular endothelial growth factor (for example the anti-vascular endothelial cell growth factor antibody bevacizumab, a compound disclosed in WO 97/22596, WO 97/30035, WO 97/32856 or WO 98/13354), or a compound that works by another mechanism (for example linomide, an inhibitor of integrin (xvp3 function or an angiostatin);
(vi) a vascular damaging agent such as combretastatin A4, or a compound disclosed in WO 99/02166, WO 00/40529, WO 00/41669, WO O1/92224, WO 02/04434 or WO 02/08213;
(vii) an agent used in antisense therapy, for example one directed to one of the targets listed above, such as ISIS 2503, an anti-ras antisense;
(viii) an agent used in a gene therapy approach, for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; or
(ix) an agent used in an immunotherapeutic approach, for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of CRTh2 receptor activity is beneficial.

In the context of the present specification, the term "therapy" also includes "prophylaxis" unless there are specific indications to the contrary. The terms "therapeutic" and "therapeutically" should be construed accordingly.

The invention still further provides a method of treating diseases mediated by PGD2 or its metabolites wherein the prostanoid binds to its receptor (especially CRTh2) receptor, which comprises administering to a patient a therapeutically effective amount of a compound of formula (I), or a pharmaceutically acceptable salt, solvate or prodrug thereof, as hereinbefore defined.

The invention also provides a method of treating an inflammatory disease, especially psoriasis, in a patient suffering from, or at risk of, said disease, which comprises administering to the patient a therapeutically effective amount of a compound of formula (I), or a pharmaceutically acceptable salt or solvate thereof, as hereinbefore defined.

For the above-mentioned therapeutic uses the dosage administered will, of course, vary with the compound employed, the mode of administration, the treatment desired and the disorder indicated.

For the above-mentioned therapeutic uses the dosage administered will, of course, vary with the compound employed, the mode of administration, the treatment desired and the disorder indicated.

The compound of formula (I), prodrugs and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as herein before defined, in association with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compound of the invention is administered orally.

The invention will now be illustrated by the following non-limiting examples in which, unless stated otherwise:
(i) when given, ¹H NMR data is quoted in the form of delta values for major diagnostic protons, given in parts per million (ppm) relative to tetramethylsilane (TMS) as an internal standard;
(ii) mass spectra (MS): generally only ions which indicate the parent mass are reported; (MM = MultiMode);
(iii) the title compounds of the examples and methods were named using the ACD/name and ACD/name batch (version 6.0) from Advanced Chemical Development Inc, Canada;
(iv) unless stated otherwise, reverse phase HPLC (RPHPLC) was conducted using a Symmetry, NovaPak or Ex-Terra reverse phase silica column;
(v) solvents were dried with MgSO₄ or Na₂SO₄;
(vi) reactions are carried out at RT unless otherwise stated;
(vii) the following abbreviations are used:
   - aq.: Aqueous
   - BBr₃: Boron tribromide
   - Boc: *tert*-Butoxycarbonyl
   - BuLi: Butyl lithium
   - CDI: 1,1'-Carbonyldiimidazole
   - Conc.: Concentrated
   - MCPBA: 3-Chloroperoxybenzoic acid (Aldrich 77% max)
   - DCM: Dichloromethane
   - DMF: *N*,*N*-dimethylformamide
   - DMPU: 1,3-Dimethyl-3,4,5,6-tetrahydro-2(1*H*)-pyrimidinone
   - DMSO: Dimethyl sulfoxide
   - Ether: Ether
   - EtOAc: Ethyl acetate
   - HATU: *O*-(7-Azabenzotriazol-1-yl)-1,1,3,3-tetramethyluronium hexafluorophosphate
   - HBr: Hydrogen bromide
   - HCl: Hydrochloric acid
   - HMPA: Hexamethylphosphoramide
   - HPLC: High performance liquid chromotography
   - K₂CO₃: Potassium carbonate
   - MeI: Methyl iodide
   - MgSO₄: Anhydrous magnesium sulphate
   - Na₂CO₃: Sodium carbonate
   - NaHCO₃: Sodium hydrogen carbonate
   - NaOH: Sodium hydroxide
   - Na₂SO₄: Sodium sulphate
   - NBS: N-Bromosuccinimide
   - NMP: *N*-Methylpyrrolidine
   - NMR: Nuclear magnetic resonance
   - Pd(OAc)₂: Palladium (II) acetate
   - PPh₃: Triphenylphosphine
   - PyBOP: (Benzotriazol-1-yloxy)tripyrrolidino-phosphonium hexafluorophosphate
   - RT: RT
   - THF: Tetrahydrofuran

### Reference Example 2

### 5-(2-{4-chloro-2-[2-chloro-4-(methylsulfonyl)phenoxy]phenyl}ethyl)-1H-tetrazole

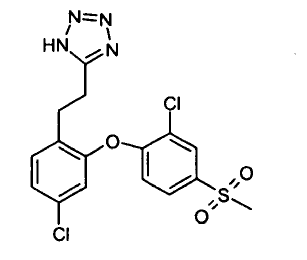

### (i) (2E)-3-(4-chloro-2-methoxyphenyl)acrylonitrile

1-Bromo-4-chloro-2-methoxybenzene (1 g), acrylonitrile (0.45 ml), DMF (4 ml), palladium acetate (0.1 g), triphenylphosphine (0.24 g) and triethylamine (0.9 g) were charged to a flask and heated at 90 °C for 4 days. The reaction mixture was partitioned between ethyl acetate and water. The organic layer was separated, dried (MgSO₄) and then evaporated under reduced pressure. The residue was purified using flash column chromatography (eluent 1:4 ethyl acetate/hexane) to give the subtitle compound as a yellow oil (0.31 g).

### (ii) 3-(4-chloro-2-methoxyphenyl)propanenitrile

The product of step (i) (0.3 g) was dissolved in ethyl acetate (10 ml). Platinum on charcoal (5 %, 0.1 g) was added and the reaction mixture was stirred under 1 bar hydrogen overnight. The reaction mixture was filtered to remove catalyst, the filtrate was evaporated under reduced pressure, then purified using flash column chromatography (eluent 1:4 ethyl acetate/hexane) to give the subtitle compound (0.25 g).
¹H NMR CDCl₃: δ 7.1 (1H, d), 6.9 (1H, dd), 6.85 (1H, d), 3.83 (3H, s), 2.91 (2H, t), 2.6 (2H, t).

### (iii) 3-(4-chloro-2-hydroxyphenyl)propanenitrile

BBr₃ (1M in DCM, 3.5 ml) was added dropwise to a solution of the product from step (ii) (0.23 g) in DCM (10 ml) and stirred overnight at RT. The reaction was quenched with ice/water. The organic layer was separated, washed with brine, dried (MgSO₄) then evaporated under reduced pressure.
MS: ESI(-ve) 180 (M-H).

### (iv) 3-{4-chloro-2-[2-chloro-4-(methylsulfonyl)phenoxy]phenyl}propanenitrile

The product from step (iii) (0.2 g), 2-chloro-1-fluoro-4-(methylsulfonyl)benzene (0.23 g), potassium carbonate (0.18 g) and DMF (5 ml) were charged to a flask stirred for 24 h. The reaction was quenched with water, extracted with ethyl acetate (x 2). The organic extracts were dried (MgSO₄) then evaporated under reduced pressure. The residue was purified using flash column chromatography (eluent 1:4 ethyl acetate/hexane) to give the subtitle compound (0.15 g).
¹H NMR CDCl₃: δ 8.1 (1H, d), 7.82 (1H, dd), 7.23 (1H, d), 7.21 (1H, dd), 7.06 (1H, d), 6.83 (1H, d), 3.1 (3H, s), 2.9 (2H, t), 2.71 (2H, t).

### (v) 5-(2-{4-chloro-2-[2-chloro-4-(methylsulfonyl)phenoxy]phenyl}ethyl)-1H-tetrazole

Sodium azide (0.07 g) was added to a stirred solution of the product of step (iv) (0.12 g), ammonium chloride (0.04 g) in DMF (5 ml) and heated at 100 °C for 4 days. The reaction mixture was partitioned between ethyl acetate and water. The organic layer was separated, washed (water), dried (MgSO₄) and then evaporated under reduced pressure. The residue was purified by RPHPLC to give the title compound (0.02 g).
¹H NMR DMSO-d6: δ 8.15 (1H, d), 7.85 (1H, dd), 7.4 (1H, d), 7.3 (1H, dd), 7.18-7.1 (2H, m), 3.37 (3H, s), 3.23 (2H, t) and 3.02 (2H, t).
MS: APCI(-ve) 411 (M-H).

### Reference Example 13

### 5-[3-{2-fluoro-4-[(4-fluorobenzyl)sulfonyl]phenoxy}-5-(trifluoromethyl)benzyl]-1H-tetrazole

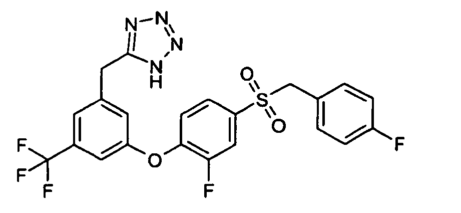

### (i) 3-methoxy-5-(trifluoromethyl)benzoic acid

Sodium methoxide (13.0 g) was added to a stirred solution of 3-fluoro-5-trifluoromethyl benzoic acid (25.0 g) in DMPU (200 ml) and heated at 120 °C overnight. The reaction was poured onto 2M HCl (800 ml). The resulting solid formed was filtered and washed with water, then dried in vacuo to give the subtitle compound (23.0 g).
¹H NMR DMSO-d6: δ□7.75 (1H, s), 7.70 (1H, s), 7.51 (1H, s), 3.90 (3H, s).

### (ii) [3-methoxy-5-(trifluoromethyl)phenyl]methanol

Lithium aluminium hydride (1M in THF, 113 ml) was added dropwise to a stirred solution of the product of step (i) (22.7 g) in THF (100 ml) at 5 °C and stirred for 2 h at RT. The reaction was cooled and water added (4.3 ml) followed by 15% NaOH (4.3 ml). The resultant precipitate was removed by filtration, washed with THF and the filtrates evaporated to an oil which was purified by flash column chromatography (eluent 4:1 isohexane/ethyl acetate) to give the subtitle compound (12.0 g).
¹H NMR DMSO-d6: δ□7.24 (1H, s), 7.17 (1 H, s), 7.09 (1H, s), 5.40 (1 H, t), 4.55 (2H, t), 3.83 (3H, s).

### (iii) [3-methoxy-5-(trifluoromethyl)phenyl]acetonitrile

Triethylamine (2.04 ml) was added to a solution of the product of step (ii) (3.02 g) in DCM (30 ml) and cooled to 0 °C before adding methane sulfonyl chloride (1.13 ml). The reaction mixture was stirred for 2 h at RT. The reaction mixture was diluted with water, extracted with DCM then dried (MgSO₄) and evaporated under reduced pressure to give an oil. The oil was dissolved in DMF (20 ml), sodium cyanide (1.07 g) was added and stirred at 100 °C for 2 h. The reaction mixture was diluted with water, extracted with ether, dried (MgSO₄) and evaporated under reduced pressure. The residue was purified by flash column chromatography (eluent ether) to give the subtitle compound (1.9 g).
¹H NMR CDCl₃-d6: δ□7.16-7.06 (3H, m), 3.87 (3H, s), 3.78 (2H, s).

### (iv) [3-hydroxy-5-(trifluoromethyl)phenyl]acetic acid

The product of step (iii) (0.53 g), tetrabutylammonium chloride (0.123 g) and 48 % aqueous HBr (5 ml) in glacial acetic acid (5 ml) were charged to a flask and heated at 125 °C for 36 h. The reaction mixture was partitioned between water and ethyl acetate, the organics were separated then dried (MgSO₄) and evaporated under reduced pressure to give the subtitle compound (0.35 g).
¹H NMR DMSO-d6: δ□12.33 (1H, s), 9.87 (1H, s), 6.75-6.65 (3H, m), 3.50 (2H, s).

### (v) 3,4-Difluorophenyl 4-fluorobenzyl sulfone

A solution of 3,4-difluorobenzenethiol (1.0 g), 1-(bromomethyl)-4-fluorobenzene (1.28 g), potassium carbonate (0.94 g) and DMF (10 ml) were charged to a flask and stirred for 2 h. The solution was partitioned between ether and water. The organic extracts were dried (MgSO₄) and evaporated under reduced pressure to give an oil which was dissolved in DCM (20 ml) and MCPBA (2.94 g) added. The mixture was stirred overnight, diluted with DCM and washed with aqueous sodium metabisulfite then aqueous NaHCO₃. The organic phase was dried (MgSO₄) and evaporated under reduced pressure, to give the subtitle compound, (1.4 g).
¹H NMR CDCl₃-d6: δ□ 7.51-7.46 (1H, m), 7.41-7.37 (1H, m), 7.29-7.23 (1H, m), 7.12-7.00 (4H, m), 4.29 (2H, s).

### (vi) [3-{2-fluoro-4-[(4-fluorobenzyl)sulfonyl]phenoxy}-5-(trifluoromethyl)phenyl]acetic acid

The product of step (iv) (200 mg), the product of step (v) (310 mg), cesium carbonate (590 mg) and NMP (10 ml) were charged to a flask and heated for 8 h at 80 °C. The solution was acidified (1M HCl) and extracted with ethyl acetate. The organic extracts were dried (MgSO₄) and evaporated under reduced pressure. The residue was purified by RPHPLC to give the title compound (0.16 g).
¹H NMR DMSO-d6: δ 7.80-7.76 (1H, d) 7.53-7.48 (2H, m), 7.34-7.12 (7H, m), 4.76 (2H, s), 3.74 (2H, s).

### (vi) 5-[3-{2-fluoro-4-[(4-fluorobenzyl)sulfonyl]phenoxy}-5-(trifluoromethyl)benzyl]-1H-tetrazole

The product of step (vi) (110 mg) was disolved in DCM (2 ml) and CDI (40 mg) added. After stirring for 1 h further CDI (15 mg) was added. It was stirred for a further 1h and then added dropwise to ammonia solution (10 ml of 7N in MeOH) and the reaction stirred for 6 h at RT. Volatiles were removed to give a crude primary amide. This was dissolved in DCM (2 ml) and pivaloyl chloride (0.14 ml) and pyridine (0.11 ml) were added and the reaction heated at reflux for 72 h. After cooling to RT it was poured into ethyl acetate and aqueous 2M HCl and the organic layer separated. The organic phase was washed with aqueous 2M HCl, then aqueous NaHCO₃, dried (MgSO₄) and evaporated under reduced pressure to give a crude nitrile. This was dissolved in toluene (1 ml), flushed with nitrogen and TMS-azide (0.1 ml) and TBAF (0.25 ml of 1M in THF) added and the reaction heated at 110 °C for 6 h. After cooling to RT, ethyl acetate and aqueous 2M HCl were added and the organic layer separated. The organic phase was purified by passage through NH2 resin eluting with ethyl acetate, MeCN, MeOH and DCM followed by 20% AcOH in DCM. The acid fractions were collected and evaporated under reduced pressure. Final purification by RPHPLC gave the title compound as a white solid (0.16 g).
1H NMR DMSO-d6: δ 7.78 (1H, d), 7.57 (1H, s), 7.49 (1H, d), 7.39 (2H, s), 7.32 (1H, t), 7.27 - 7.11 (4H, m), 4.77 (2H, s), 4.39 (2H, s).
MS: APCI(-ve): 509 (M-H).

### Reference Example 14

### 5-{3-chloro-5-[2-chloro-4-(ethylsulfonyl)phenoxy]benzyl}-1H-tetrazole

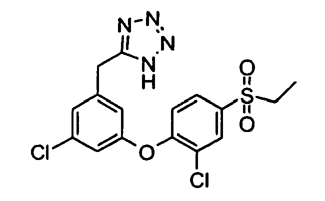

### (i) 3-chloro-5-methoxybenzoic acid

Sodium methoxide (22 g) was added to a stirred solution of 3,5-dichlorobenzoic acid (25 g) in HMPA (140 ml) and heated at 170 °C for 48 h. The reaction was poured onto 1 M HCl (400 ml) and the mixture poured onto ice. The resulting solid formed was filtered and washed with water, then dried in vacuo to give the subtitle compound (15.2 g).
¹H NMR DMSO-d6: δ 13.34 (1H, s), 7.46 (1H, s), 7.38 (1H, s), 7.30 (1H, s), 3.77 (3H, s).

### (ii) (3-chloro-5-methoxyphenyl)methanol

Lithium aluminium hydride (1M in THF, 90 ml) was added dropwise to a stirred solution of the product of step (i) (15.2 g) in THF (80 ml) and stirred for 2 h. The reaction was diluted with 2 M HCl and extracted with ethyl acetate. The organic layer was washed with aqueous sodium hydrogen carbonate, dried (MgSO₄) and evaporated under reduced pressure to give the subtitle compound (9.8 g).
¹H NMR CDCl₃-d6: δ 6.93 (1H, s), 6.82-6.71 (2H, m), 4.63 (2H, s), 3.79 (3H, s).

### (iii) 1-chloro-3-(chloromethyl)-5-methoxybenzene

Thionyl chloride (5 ml) was added to a solution of the product of step (ii) (9.8 g) in DCM (100 ml) at 5 °C. DMF (0.4 ml) was added and the mixture stirred at RT for 20 h then washed (water then brine) and the organics dried (MgSO₄) and evaporated under reduced pressure to give the subtitle compound as a brown oil which solidified on standing (9.6 g). ¹H NMR CDCl₃-d6: δ 6.99 (1H, t), 6.85 (1H, t), 6.82 (1H, t), 4.50 (2H, s), 3.81 (3H, s).

### (iv) (3-chloro-5-methoxyphenyl)acetonitrile

Sodium cyanide (2.9 g) was added to a solution of the product of step (iii) (9.6 g) in DMF (100 ml). The mixture was stirred at RT for 72 h then poured onto water (1200 ml) and the organics extracted into ether then washed (brine), dried (MgSO₄) and evaporated under reduced pressure to give the subtitle compound as a yellow oil which solidified on standing (6.5 g).
¹H NMR CDCl₃-d6: δ 6.91 (1H, s), 6.86 (1H, d), 6.77 (1H, s), 3.81 (3H, s), 3.69 (2H, s).

### (v) (3-chloro-5-hydroxyphenyl)acetonitrile

Boron tribromide (1M in DCM, 20 ml) was added dropwise to a solution of the product of step (iv) (1.8 g) in DCM (60 ml) at 5 °C. Mixture warmed to RT and stirred for 16 h. Further boron tribromide was then added (10 ml), the mixture was stirred for a further 24 h then water was added (caution) and the mixture extracted with DCM. The organics were dried (MgSO₄) and evaporated under reduced pressure to give the subtitle compound as a yellow solid (1.2 g).
MS: MM(-ve): 166 (M-H).

### (v) 3-chloro-5-(1H-tetrazol-5-ylmethyl)phenol

Trimethylsilylazide (1.4 ml) and TBAF (3.5 ml) were added to a solution of the product of step (iv) (1.2 g) in toluene (15 ml). The mixture was heated to 120 °C and stirred for 22 h. 2M HCl and ethyl acetate were added, the organics dried (MgSO₄) and evaporated under reduced pressure to give the subtitle compound as a brown solid (1.4 g) used as crude without further purification.
MS: MM(-ve): 209 (M-H).

### (vi) 5-{3-chloro-5-[2-chloro-4-(ethylsulfonyl)phenoxy]benzyl}-1H-tetrazole

A solution of the product of step (v) (230 mg), 2-chloro-4-(ethylsulfonyl)-1-fluorobenzene (240 mg) and cesium carbonate (1.1 g) in NMP (3 ml) were heated at 80°C for 4 h. The mixture was cooled, diluted with 1M HCl and extracted with EtOAc. The organics were dried (MgSO₄) and evaporated under reduced pressure and the residue purified by RPHPLC to give the title compound as a white solid, yield 170 mg.
1H NMR DMSO-d6: δ 8.09 (1H, d), 7.84 (1H, dd), 7.29-7.27 (2H, m), 7.23 (1H, t), 7.10 (1H, s), 4.35 (2H, s), 3.37 (2H, q), 1.13 (3H, t).
MS: MM(+ve): 413 (M+H).

### Reference Example 16

### 5-{3-chloro-5-[2-chloro-4-(phenylsulfonyl)phenoxy]benzy}-1H-tetrazole

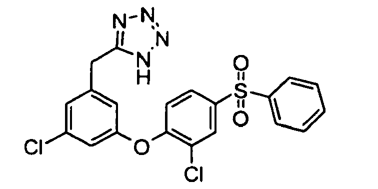

### (i) 3-chloro-4-fluorophenyl phenyl sulfone

Ferric chloride (0.47 g), was added to a stirred mixture of 3-chloro-4-fluorosulfonyl chloride (1.5 g) and benzene (10 ml). The reaction mixture was then heated to reflux for 18 h, then allowed to cool to RT. The solvent was evaporated under reduced pressure and the residue was partitioned between DCM and NaHCO₃ (aq), then extracted with DCM (x 2). The combined organic extracts were washed with brine, dried (MgSO₄) and evaporated under reduced pressure. The residual solid was recrystallised from ethanol to give the subtitle compound as buff coloured crystals (1.1g).
¹H NMR CDCl₃: δ 8.02-7.31 (8H, m).

### (ii) 5-{3-chloro-5-[2-chloro-4-(phenylsulfonyl)phenoxy]benzyl}-1H-tetrazole

The title compound was synthesised by the method of example 14 step (vi) using the product of example 14 step (v) and the product of step (i) to give a white solid.
1H NMR DMSO-d6: δ 8.19 (1H, d), 8.03 (1H, s), 8.01 (1H, t), 7.91 (1 H, dd), 7.72 (1H, tt), 7.65 (2H, tt), 7.28 (1H, t), 7.24 (1H, t), 7.16 (1H, d), 7.10 (1H, t), 4.32 (2H, s).
MS: MM(+ve): 461 (M+H).

### Reference Example 17

### 5-(3-chloro-5-{2-chloro-4-[(4-fluorobenzyl)sulfonyl]phenoxy}benzyl)-1H-tetrazole

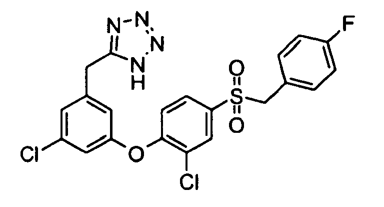

### (i) 2-chloro-1-fluoro4-[(4-fluorobenzyl)sulfonyl]benzene

A solution of 3-chloro-4-fluorobenzenethiol (1.0 g), 1-(bromomethyl)-4-fluorobenzene (1.15 g) and potassium carbonate (0.85 g) in DMF (10 ml) was stirred overnight at RT then diluted with water, extracted with ether, and the organics dried (MgSO₄) and evaporated under reduced pressure. The resulting oil was dissolved in DCM (10 ml) and MCPBA (1.2 g) added and stirred at RT overnight. The solution was then washed with aqueous sodium metabisulphite and aqueous NaHCO₃, dried (MgSO₄) and evaporated under reduced pressure to give an oil which was purified by flash column chromatography (eluent 3:2 i-hexane / ether) to give the subtitle compound as a white solid (1.3 g).
¹H NMR CDCl₃: δ 7.74-7.71 (1H, d), 7.53-7.49 (1H, m), 7.28-7.20 (2H, m), 7.08-7.06 (1H, m), 6.92-6.86 (2H, m), 4.31 (2H, s).

### (ii) 5-(3-chloro-5-{2-chloro-4-[(4-fluorobenzyl)sulfonyl]phenoxy}benzyl)-1H-tetrazole

The title compound was synthesised by the method of example 14 step (vi) using the product of example 14 step (v) and the product of step (i) to give a white solid.
1H NMR DMSO-d6: δ 7.90 (1H, d), 7.60 (1H, dd), 7.28-7.14 (7H, m), 7.06 (1H, s), 4.77 (2H, s), 4.33 (2H, s).
MS: MM(+ve): 491 (M+H).

### Reference Example 18

### 2-[3-chloro-5-(1H-tetrazol-5-ylmethyl)phenoxy]-5-(phenylsulfonyl)benzonitrile

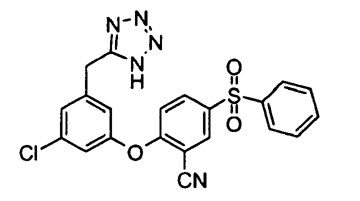

### (i) 2-chloro-5-(phenylsulfonyl)benzonitrile

A solution of 5-amino-2-chlorobenzonitrile (6.6 g), diphenyldisulphide (11.0 g) and isoamylnitrile (10 ml) in acetonitrile (100 ml) was heated at 60 °C for 6 h then evaporated under reduced pressure. The residue was purified by flash column chromatography (eluent: iso-hexane to 1% EtOAc in iso-hexane) and the resulting solid dissolved in DCM. MCPBA (8.0 g) was added portionwise and the mixture stirred for 2 h, filtered and the filtrate washed with aqueous sodium metabisulphite, aqueous NaHCO₃ then water and the organics dried (MgSO₄) and evaporated under reduced pressure to give the subtitle compound (2.9 g).
¹H NMR CDCl₃: δ 8.22 (1H, s), 8.08 (1H, d), 7.95 (2H, d), 7.68-7.54 (4H, m).

### (ii) 2-[3-chloro-5-(1H-tetrazol-5-ylmethyl)phenoxy]-5-(phenylsulfonyl)benzonitrile

The title compound was synthesised by the method of example 14 step (vi) using the product of example 14 step (v) and the product of step (i) to give a white solid.
1H NMR DMSO-d6: δ 8.60 (1H, d), 8.17 (1H, ddd), 8.03 (2H, d), 7.75-7.62 (3H, m), 7.44 (1H, t), 7.38 (1H, s), 7.25 (1H, s), 7.09 (1H, d), 3.31 (2H, s).
MS: MM(+ve): 452 (M+H).

### Reference Example 20

### {3-chloro-4-[3-chloro-5-(1H-tetrazol-5-ylmethyl)phenoxy]phenyl}(phenyl)methanone

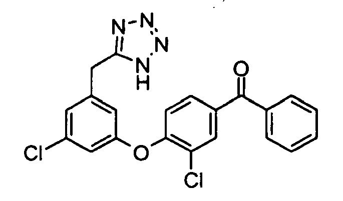

### (i) (3-chloro-4-fluorophenyl)(phenyl)methanone

3-chloro-4-fluorobenzoyl chloride (1.0 g), benzene (2 ml) and ferric chloride (0.28 g) were heated to reflux for 16 h then diluted with water, extracted with ethyl acetate and the organics dried (MgSO₄) and evaporated under reduced pressure to give the subtitle compound as a brown solid (0.8 g).
¹H NMR CDCl₃: δ 7.91-7.88 (1H, d), 7.77-7.48 (6H, m), 7.28-7.22 (1H, t).

### (ii) {3-chloro-4-[3-chloro-5-(1H-tetrazol-5-ylmethyl)phenoxy] phenyl}(phenyl)methanone

The title compound was synthesised by the method of example 14 step (vi) using the product of example 14 step (v) and the product of step (i) to give a white solid.
1H NMR DMSO-d6: δ 7.93 (1H, s), 7.79 (1H, s), 7.77 (1H, d), 7.73 (1H, d), 7.72-7.69 (1H, m), 7.59 (2H, t), 7.26-7.22 (2H, m), 7.17 (1H, t), 7.09 (1H, t), 4.36 (2H, s).
MS: MM(+ve): 425 (M+H).

### Reference Example 21

### 2-{2-[2-chloro-3-(methylsulfonyl)benzyl]-4-fluorophenoxy}-N-(methylsulfonyl)acetamide

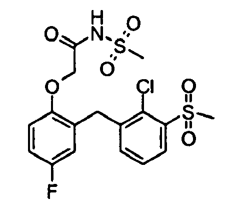

### (i) 2-chloro-1-(5-fluoro-2-methoxybenzyl)-3-(methylsulfonyl)benzene

A solution of 1-(bromomethyl)-2-chloro-3-(methylsulfonyl)benzene (1.0 g) and (5-fluoro-2-methoxyphenyl)boronic acid (0.6 g) in dioxane (20 ml) was treated with bisdiphenylphosphinoferrocene palladium (II) (0.13 g) and cesium fluoride (1.13 g) and heated to 80 °C for 1 h. The mixture was diluted with water, extracted with ether and the organics dried (MgSO₄) and evaporated under reduced pressure to give an oil which was purified by flash column chromatography (eluent 2:1 ether / isohexane) to give a white solid (0.9 g).
MS: APCI(-ve): 327 (M-H).

### (ii) 2-[2-chloro-3-(methylsulfonyl)benzyl]-4-fluorophenol

The product of step (i) in 48% aqueous HBr (20 ml) was treated with acetic acid (5 ml) and heated at 100 °C for 8 h. The mixture was then evaporated under reduced pressure to give a cream solid (0.77 g).
MS: APCI(-ve): 313 (M-H).

### (iii) {2-[2-chloro-3-(methylsulfonyl)benzyl]-4-fluorophenoxy}acetic acid

The product of step (ii) (0.2 g), tert-butyl bromoacetate (0.10 ml) and K₂CO₃ (0.09 g) in DMF (10 ml) were stirred at RT for 2 h then water was added and the mixture extracted with ethyl acetate. The organics were dried (MgSO₄) and evaporated under reduced pressure to an oil which was dissolved in 1:1 TFA / DCM (20 ml) and stirred at RT for 2 h. The mixture was evaporated under reduced pressure and the resulting oil purified by RPHPLC to give the subtitle compound as a white solid (0.18 g).
MS: APCI(-ve): 371 (M-H).

### (iv) 2-{2-[2-chloro-3-(methylsulfonyl)benzyl]-4-fluorophenoxy}-N-(methylsulfonyl) acetamide

The title compound was prepared by the method of example 3 step (vi) using the product of step (iii) and methanesulfonamide.
1H NMR DMSO-d6: δ 12.01 (1H, s), 7.99 (1H, s), 7.81 (1H, d), 7.48 (1 H, d), 7.10 (1H, t), 6.96-6.92 (2H, m), 4.72 (2H, s), 4.17 (2H, s), 3.25 (6H, s).
MS: MM(-ve): 448 (M-H).

### Example 1

### 2-[2-[2-chloro-4-(methylsulfonyl)phenoxy]-4-(trifluoromethyl)phenoxy]-N-(methylsulfonyl)acetamide

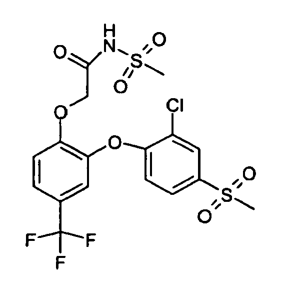

Oxalyl chloride (10 ml) was added to a stirred solution of [2-[2-chloro-4-(methylsulfonyl)phenoxy]-4-(trifluoromethyl)phenoxy]acetic acid (W02005018529) (0.4 g) in DCM. DMF (cat.) was added. The reaction was evaporated under reduced pressure, then azeotroped with toluene. The residue was dissolved in DCM (20 ml), Hunigs base (0.35 ml) was added followed by methanesulfonamide (0.19 g) and stirred for 20 h. The reaction was quenched with water. The organic layer was dried (MgSO₄) and then evaporated under reduced pressure. The residue was purified by RPHPLC to give the title compound (0.05 g).
¹H NMR DMSO-d6: δ 8.07 (1H, s), 7.77 (1H, d), 7.66 (1H, d), 7.60 (1H, s), 7.21 (1H, d), 7.09 (1H, d), 6.95 1H, s), 4.56 (2H, s), 3.25 (3H, s), 2.87 (3H, s).
MS: ESI(-ve) 500 (M-H)

### Example 2

### 2-14-chloro-3-[2-chloro-4-(methylsulfonyl)phenoxy]phenyl}-N-(methylsulfonyl) acetamide

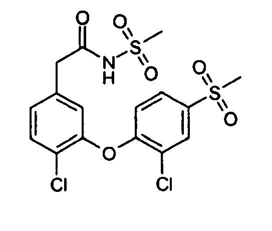

### (i) 4-(Bromomethyl)-1-chloro-2-methoxybenzene

2-Chloro-5-methylphenol (20 g), K₂CO₃ (30 g), acetone (200 ml) and methyl iodide (9.4 ml) were charged to a flask and stirred for 24 h. The solvent was evaporated under reduced pressure and the residue partitioned between ether and water. The organics were separated, washed with 2 M NaOH then water, dried (MgSO₄) and evaporated under reduced pressure. The residue was dissolved in ethyl acetate, then NBS (25 g) and benzoyl peroxide (0.5 g) were added and the reaction mixture irradiated with a halogen lamp for 3 h. The solvent was evaporated under reduced pressure and the residue was purified by flash column chromatography (eluent isohexane) to give the subtitle compound (30 g) used directly without further purification or characterisation.

### (ii) (4-Chloro-3-methoxyphenyl)acetic acid

The product from step (i) DMF (200 ml) and sodium cyanide (20 g) were charged to a flask and stirred for 2 h at RT. The residue was partitioned between ether and water; the organics were separated, washed with water, dried (MgSO₄) and evaporated under reduced pressure. Potassium hydroxide (40 g in water) was added and the mixture heated at reflux for 24 h. The reaction mixture was cooled to RT and extracted with ether. The aqueous layer was acidified to pH 1 with concentrated HCl and extracted with ethyl acetate. The organic layer was washed with water, dried (MgSO₄) and evaporated under reduced pressure. The residue was triturated with isohexane/ether, and then filtered to give the subtitle compound (12.2 g).

### (iii) (4-Chloro-3-hydroxyphenyl)acetic acid

The product from step (ii) (12.2 g), HBr (48 % aq.) (10 ml) and acetic acid (10 ml) were charged to a flask and heated at reflux for 24 h, cooled then evaporated under reduced pressureevaporated under reduced pressure. The residue was triturated with ether/isohexane, and then filtered to give the subtitle compound (10.6 g).
¹H NMR CDCl₃-d6: δ 7.32 (1H, d), 6.85 (1H, s), 6.82 (1H, d), 3.9 (3H, s), 3.63 (2H, s).

### (iv) Ethyl (4-chloro-3-hydroxyphenyl)acetate

The product of step (iii) (4 g) was added to a solution of acetyl chloride (10 ml) in ethanol (40 ml). The mixture was stirred for 1 h at RT then evaporated under reduced pressure. The residue was purified by flash column chromatography (eluent 2:1 hexane/ethyl acetate) to give the subtitle compound (4.4 g).

### (v) Ethyl {4-chloro-3-[2-chloro-4-(methylsulfonyl)phenoxy]phenyl}acetate

The product from step (iv) (4.4 g), 3-chloro-4-fluorophenyl methyl sulfone (4.27 g), cesium carbonate (6.5 g) and NMP (40 ml) were charged to a flask and stirred at 90 °C for 2 h. The reaction was diluted with water, extracted (ethyl acetate), dried (MgSO₄) and evaporated under reduced pressure. The residue was purified by flash column chromatography (eluent 2:1 isohexane/ ether) to give the subtitle compound (3.6 g).
MS: ESI-ve 401 (M-H).

### (vi) {4-Chloro-3-[2-chloro-4-(methylsulfonyl)phenoxy]phenyl}acetic acid

Sodium hydroxide (0.72 g) in water (40 ml) was added to the product from step (v) (3.6 g) in THF (40 ml) and stirred at RT overnight. The reaction was quenched with 2M HCl extracted (ethyl acetate), dried (MgSO₄) and evaporated under reduced pressure. The residue was recrystallised from ethyl acetate/isohexane to give the subtitle compound (2.6 g).
¹H NMR DMSO-d6: δ 12.46 (1H, s), 8.15-8.14 (1H, s), 7.84 (1H, d), 7.63-7.59 (1H, d), 7.28-7.24 (2H, m), 6.93 (1H, d), 3.64 (2H, s), 3.27 (3H, s).
MS: ESI-ve 372 (M-H).

### (vii) 2-{4-chloro-3-[2-chloro-4-(methylsulfonyl)phenoxy]phenyl}-N-(methylsulfonyl) acetamide

Hunigs base (0.87 ml) was added to a stirred suspension of the product from step (vi) (0.38 g), PyBOP (0.78 g) and methanesulfonamide (0.11 g) in DCM (7.7 ml). The mixture was stirred at RT for 2 days then washed (water), the organics separated then evaporated under reduced pressure. The residue was purified by RPHPLC. The resulting solid was dissolved in acetonitrile and a solution of sodium hydroxide (1 eq.) in methanol was added and stirred for 1 h. The reaction mixture was evaporated under reduced pressure to give the title compound as a hygroscopic white solid (0.20 g).
¹H NMR DMSO-d6: δ 8.15 (1H, d), 7.85 (1H, dd), 7.58 (1H, d), 7.24 (1H, d), 7.22 (1H, s), 6.97 (1H, d), 3.38 (2H, s), 3.29 (3H, s), 2.78 (3H, s).
MS: APCI(-ve) 450 (M-H).

### Example 3

### 2-{3-chloro-5-[2-chloro-4-(methylsulfonyl)phenoxy]phenyl}-N-(methylsulfonyl) acetamide

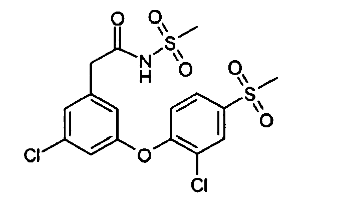

### (i) 3-chloro-5-methoxybenzoic acid

Sodium methoxide (25% wt., 7 ml) was added to a stirred solution of 3,5-dichlorobenzoic acid (2 g) in DMPU (10ml) and heated at 170 °C for 5 days. The reaction was poured onto 1M HCl (50 ml). The resulting solid formed was filtered and washed with water, then dried in vacuo to give the subtitle compound (0.8 g).
¹H NMR DMSO-d6: δ 13.34 (1H, s), 7.46 (1H, s), 7.38 (1H, s), 7.3 (1H, s), 3.77 (3H, s).

### (ii) (3-chloro-5-methoxyphenyl)methanol

Lithium aluminium hydride (1M in THF, 8.76 ml) was added dropwise to a stirred solution of the product of step (i) (1.63 g) in THF (40 ml) and stirred for 2 h. The reaction was diluted with 2 M HCl and extracted with ethyl acetate. The organic layer was washed with aqueous sodium hydrogen carbonate, dried (MgSO₄) and evaporated under reduced pressure to give the subtitle compound (1.53 g).
¹H NMR CDCl₃-d6: δ 6.93 (1H, s), 6.82-6.71 (2H, m), 4.63 (2H, s), 3.79 (3H, s).

### (iii) (3-chloro-5-methoxyphenyl)acetonitrile

Phosphorous tribromide (0.28 ml) was added to a solution of the product of step (ii) (1.55 g) in ether (20 ml) at 0 °C, then stirred for 30 min. The reaction mixture was partitioned between ether and aqueous sodium hydrogen carbonate, the organics were separated then dried (MgSO₄) and evaporated under reduced pressure. The residue was dissolved in DMF (20 ml) and sodium cyanide (0.5 g) was added. The mixture was stirred overnight then partitioned between ether and water; the organics were separated, washed with aqueous sodium hydrogen carbonate then dried (MgSO₄) and evaporated under reduced pressure. The residue was purified by flash column chromatography (eluent 1:1 ether/ isohexane) to give the subtitle compound (0.53g).
¹H NMR CDCl₃-d6: δ 6.91-6.9 (1H, m), 6.86-6.85 (1H, m), 6.77-6.76 (1H, m), 3.79 (3H, s), 3.69 (2H, s).

### (iv) (3-chloro-5-hydroxyphenyl)acetic acid

The product of step (iii) (0.53 g), tetrabutylammonium chloride (0.123 g) and 48 % aqueous HBr (5 ml) in glacial acetic acid (5 ml) were charged to a flask and heated at 125 °C for 36 h. The reaction mixture was partitioned between water and ethyl acetate, the organics were separated then dried (MgSO₄) and evaporated under reduced pressure.
¹H NMR DMSO-d6: δ 12.33 (1H, s), 9.87 (1H, s), 6.75-6.65 (3H, m), 3.50 (2H, s).

### (v) {3-chloro-5-[2-chloro-4-(methylsulfonyl)phenoxy]phenyl}acetic acid

The product of step (iv) (0.50 g) and 3-chloro-4-fluorophenyl methyl sulfone (0.56 g), cesium carbonate (1.75 g) and NMP (20 ml) were charged to a flask and heated for 5 h at 80 °C. The solution was diluted with 1M NaOH solution and extracted with ether. The aqueous layer was then acidified (1M HCl) and extracted with ethyl acetate. The organic extracts were dried (MgSO₄) and evaporated under reduced pressure. The residue was purified by RPHPLC to give the subtitle compound (0.45 g).
¹H NMR DMSO-d6: δ 8.14 (1H, s), 7.87 (1H, d), 7.33-7.02 (4H, m), 3.62 (2H, s), 3.27 (3H, s).
MS: APCI(+ve) 392 (M+NH₄).

### (vi) 2-{3-chloro-5-[2-chloro-4-(methylsulfonyl)phenoxy]phenyl}-N-(methylsulfonyl) acetamide

Hunigs base (0.67 ml) was added to a stirred suspension of the product of step (v) (0.29 g), PyBOP (0.60 g) and methanesulfonamide (0.09 g) in DCM (6 ml). The mixture was stirred at RT for 2 h then washed (2M HCl), and the organics separated then dried (Na₂SO₄) and evaporated under reduced pressure. The residue was purified by passing through an NH₂ resin (eluent DCM, acetonitrile, methanol, DCM then 10% acetic acid in DCM). The acidic fraction was evaporated under reduced pressure to give a white solid which was triturated with toluene, filtered and dried in vacuo to give the title compound (0.12 g). ¹H NMR DMSO-d6: δ 11.95 (1H, s), 8.16 (1H, d), 7.89 (1H, dd), 7.26 (1H, d), 7.25 (1H, s), 7.19 (1H, t), 7.03 (1H, m), 3.68 (2H, s), 3.29 (3H, s), 3.23 (3H, s).
MS: APCI(-ve) 450 (M-H).

### Example 4

### 2-(3-chloro-5-{[2-fluoro-4-(phenylsulfonyl)phenyl]thio}phenyl)-N-(methylsulfonyl) acetamide

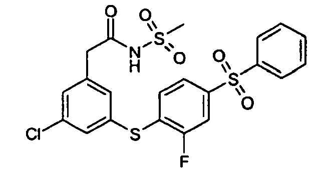

### i) methyl (3-chloro-5-hydroxyphenyl)acetate

The product from example 11 step (iv) (1.20 g) was added to a pre-formed solution of acetyl chloride (5 ml) in methanol (30 ml). The solution was stirred at RT overnight. The mixture was evaporated under reduced pressure to give a brown oil (1.70 g).
MS: APCI(-ve): 199 (M-H).

### ii) methyl (3-chloro-5-{[(dimethylamino)carbonothioyl]oxy}phenyl)acetate

The product from step (i) (1.70 g), thiocarbamoyl chloride (1.60 g), DMAP (0.103 g), triethylamine (2.40 ml) in dry dioxane (20 ml) was stirred at 100 °C for 15 h.The mixture was diluted with water, extracted with ethyl acetate, dried (MgSO₄) and evaporated under reduced pressure to an oil. The oil was purified by flash column chromatography (eluent 2:1 ether / isohexane) to give the subtitle compound (2.10 g).
1H NMR CDCl3 : δ□ 7.18-7.16 (1 H, m), 7.02-7.01 (1 H, m), 6.93-6.92 (1H, m), 3.70 (3H, s), 3.61 (2H, s), 3.44 (3H, s), 3.32 (3H, s).

### iii) methyl (3-chloro-5-mercaptophenyl)acetate

The product from step (ii) (2.10 g) in tetradecane (20 ml) was heated at 200 °C for 20 h.The reaction mixture was purified by flash column chromatography (eluent 2:1 ether / isohexane) to give the subtitle compound (1.68 g).
1H NMR CDCl3: δ□ 7.42-7.41 (1H, m), 7.31 (2H, s), 3.69 (3H, s), 3.53 (2H, s), 3.05 (6H, bm).

### iv) (3-chloro-5-mercaptophenyl)acetic acid

The product from step (iii) (1.68 g) was dissolved in methanol (10 ml) and 2M NaOH (10 ml) and stirred at RT overnight. The mixture was diluted with 2M HCl, extracted with ethyl acetate, dried (MgSO₄) and evaporated under reduced pressure to a solid (1.60 g)
1H NMR CDCl3: δ□ 7.32-7.31 (1H, s), 7.26 (1H, s), 7.08-7.07 (1H, s), 3.56 (2H, s), 2.9 (1H, s).

### v) 3,4-difluorophenyl phenyl sulfone

3,4-difluoroaniline (3.5 g), acetonitrile (60 ml), diphenyldisulfide (6 g) and isoamyl nitrite (8 ml) were charged to a flask and heated at 60 °C for 2 h then concentrated under reduced pressure. The residue was purified by flash column chromatography (eluent isohexane) to give the subtitle compound. The product (3,4-difluorophenyl phenyl sulfide) was dissolved in acetonitrile (60 ml). Water (10 ml) and oxone (20 g) were added and stirred for 72 h at RT. The reaction mixture was partitioned between ether / water, the organics were separated, washed with water, then dried (MgSO₄) and evaporated under reduced pressure. The residue was purified by flash column chromatography (eluent 5-10 % ethyl acetate/ isohexane) to give the subtitle compound (2.14 g).
¹H NMR CDCl₃-d6: δ 7.93 (2H, d), 7.81-7.71 (2H, m), 7.64-7.51 (3H, m), 7.34-7.28 (1H, m).

### vi) (3-chloro-5-{[2-fluoro-4-(phenylsulfonyl)phenyl]thio}phenyl)acetic acid

The subtitle compound was prepared by the method of reference example 14 step (vi) using the product of step (iv) and the product of step (v).
1H NMR DMSO-d6: 12.51 (1H, s), 8.00-7.91 (3H, m), 7.73-7.60 (4H, m), 7.49-7.29 (3H, m), 7.24-7.18 (1H, t), 3.65 (2H, s).
MS: APCI(-ve): 435 (M-H).

### vii) 2-(3-chloro-5-{[2-fluoro-4-(phenylsulfonyl)phenyl]thio}phenyl)-N-(methylsulfonyl) acetamide

The title compound was prepared by the method of example 3 step (vi) using the product of step (v) and methanesulfonamide.
1H NMR DMSO-d6: 11.93 (1H, s), 8.00-7.92 (3H, m), 7.75-7.61 (4H, m), 7.52 (1H, s), 7.45 (1H, s), 7.39 (1H, s), 7.25-7.21 (1H, t), 3.73 (2H, s), 3.21 (3H, s).
MS: MM(-ve): 512 (M-H).

### Example 5

### 2-[3-{2-chloro-4-[(4-fluorobenzyl)sulfonyl]nhenoxy}-5-(trifluoromethyl)phenyl]-N-(methylsulfonyl)acetamide

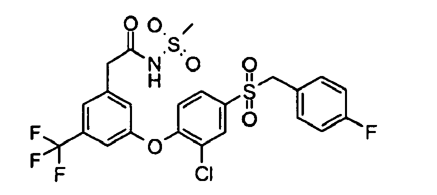

### i) [3-hydroxy-5-(trifluoromethyl)phenyl]acetic acid

The subtitle compound was prepared by the methods of example 3 steps (i) to (iv) using 3-fluoro-5-trifluoromethyl benzoic acid.
1H NMR DMSO-d6: d 7.02-6.83 (3H, m), 3.60 (2H, s).

### (ii) [3-{2-chloro-4-[(4-fluorobenzyl)sulfonyl]phenoxy}-5-(trifluoromethyl)phenyl]acetic acid

The subtitle compound was prepared by the method of reference example 14 step (vi) using the product of step (i) and the product of reference example 17 step (i).
1H NMR DMSO-d6: δ 7.92 (1H, d), 7.61 (1H, dd), 7.55 (1H, s), 7.35 (2H, d), 7.27 - 7.13 (5H, m), 4.77 (2H, s), 3.76 (2H, s).
MS: MM(-ve): 501 (M-H).

### (iii) 2-[3-{2-chloro-4-[(4-fluorobenzyl)sulfonyl]phenoxy}-5-(trifluoromethyl)phenyl]-N-(methylsulfonyl)acetamide

The title compound was prepared by the method of example 3 step (vi) using the product of step (ii) and methanesulfonamide.
1H NMR DMSO-d6: δ 11.99 (1H, s), 7.93 (1H, d), 7.61 (1H, d), 7.55 (1H, s), 7.40 (1H, s), 7.34 (1H, s), 7.28 - 7.13 (5H, m), 4.78 (2H, s), 3.80 (2H, s), 3.23 (3H, s).
MS: MM(-ve): 578 (M-H).

### Example 6

### 2-[3-[2-chloro-4-(methylsulfonyl)phenoxy]-5-(trifluoromethyl)phenyl]-N-(methylsulfonyl)acetamide

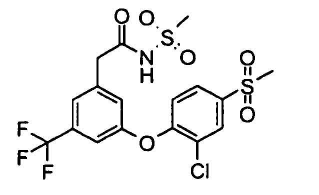

### (i) [3-[2-chloro-4-(methylsulfonyl)phenoxy]-5-(trifluoromethyl)phenyl]acetic acid

A solution of the product of example 25 step (i) (150 mg), 2-chloro-1-fluoro-4-(methyl sulfonyl)benzene (184 mg) and cesium carbonate (442mg) in DMF (1 ml) were heated at 80 °C in a microwave for 40 minutes. The mixture was cooled, diluted with 1 M HCl and extracted with ethyl acetate. The organics were washed with brine, dried (MgSO₄) and evaporated under reduced pressure to give the subtitle crude compound as an oil (185 mg). 1H NMR DMSO-d6: δ 12.52 (1H, s), 8.17 (1H, d), 7.89 (1H, dd), 7.56 (1H, s), 7.42 (1 H, s), 7.36 (1H, s), 7.26 (1H, d), 3.76 (2H, s), 3.29 (3H, s).

### (ii) 2-[3-[2-chloro-4-(methylsulfonyl)phenoxy]-5-(trifluoromethyl)phenyl]-N-(methyl sulfonyl)acetamide

The title compound was prepared by the method of example 3 step (vi) using the product from step (i) and methanesulfonamide.
1H NMR DMSO-d6: δ 8.15 (1H, d), 7.91 - 7.86 (1H, m), 7.50 (1H, s), 7.35 (1H, s), 7.31 (1H, s), 7.26 (1H, d), 3.46 (2H, s), 3.29 (3H, s), 2.78 (3H, s)
MS: MM(-ve): 484 (M-H).

### Example 7

### 2-[3-[2-cyano-4-(phenylsulfonyl)phenoxy]-5-(trifluoromethyl)phenyl]-N-(methylsulfonyl)acetamide

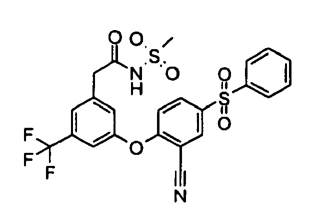

### (i) [3-[2-cyano-4-(phenylsulfonyl)phenoxy]-5-(trifluoromethyl)phenyl]acetic acid

The subtitle compound was prepared by the method of example 6 step (ii) using the product of example 5 step (i) and the product of reference example 18 step (i).
1H NMR DMSO-d6: δ 12.57 (1H, s), 8.62 (1H, d), 8.17 (1H, d), 8.03 (2H, m), 7.76 - 7.61 (5H, m), 7.54 (1H, s,), 7.04 (1H, d), 3.77 (2H, s).
MS: MM(-ve): 416 (M-CO₂H).

### (ii) 2-[3-[2-Cyano-4-(phenylsulfonyl)phenoxy]-5-(trifluoromethyl)phenyl]-N-(methyl sulfonyl)acetamide

The title compound was prepared by the method of example 3 step (vi) using the product from step (i).
1H NMR DMSO-d6: δ 8.62 (1H, s), 8.18 (1H, d), 8.03 (2H, d), 7.73 (1H, t), 7.69 - 7.64 (4H, m), 7.52 (1H, s), 7.06 (1H, d), 3.78 (2H, s), 3.17 (3H, s).
MS: MM(-ve): 537 (M-H).

### Example 8

### 2-[3-[4-(Ethylsulfonyl)-2-(trifluoromethyl)phenoxy]-5-(trifluoromethyl)phenyl]-N-(methylsulfonyl)acetamide

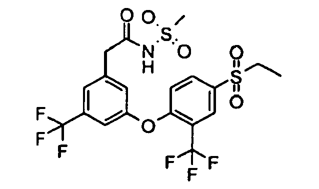

### (i) [3-[4-(ethylsulfonyl)-2-(trifluoromethyl)phenoxy]-5-(trifluoromethyl)phenyl]acetic acid

The subtitle compound was prepared by the method of example 6 step (ii) using 4-(ethyl sulfonyl)-1-fluoro-2-(trifluoromethyl)benzene.
1H NMR DMSO-d6: δ 8.20 (1H, s), 8.14 (1H, d), 7.63 (1H, s), 7.54 (1H, s), 7.47 (1H, s), 7.27 - 7.20 (1H, m), 3.78 (2H, s), 3.41 (2H, d), 1.13 (3H, t).

### (ii) 2-[3-[4-(ethylsulfonyl)-2-(trifluoromethyl)phenoxy]-5-(trifluoromethyl)phenyl]-N-(methylsulfonyl)acetamide

The title compound was prepared by the method of example 3 step (vi) using the product from step (i).
1H NMR DMSO-d6: δ 8.22 (1H, d), 8.16 (1H, m), 7.63 (1 H, s), 7.58 (1H, s), 7.48 (1H, s), 7.27 (1H, d), 3.83 (2H, s), 3.47 - 3.38 (2H, m), 3.24 (3H, s), 1.15 (3H, t).
MS: MM(-ve): 532 (M-H).

### Example 9

### 2-[3-[2-cyano-4-(phenylsulfonyl)phenoxy]-5-(trifluoromethyl)phenyl]-N-(ethylsulfonyl)acetamide

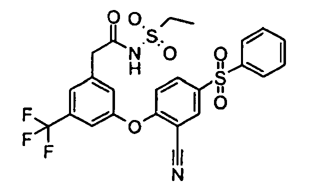

The title compound was prepared by the method of example 3 step (vi) using the product from example 27 step (i) and ethanesulfonamide.
1H NMR DMSO-d6: δ 8.59 (1H, d), 8.15 (1H, dd), 8.00 (2H, d), 7.72 - 7.58 (5H, m), 7.49 (1H, s), 7.02 (1H, d), 3.78 (2H, s), 3.36 - 3.22 (2H, m), 1.13 (3H, t)
MS: MM(-ve): 551 (M-H)

### Example 10

### 2-[3-[2-chloro-4-(phenylsulfonyl)phenoxy]-5-(trifluoromethyl)phenyl]-N-(methylsulfonyl)acetamide

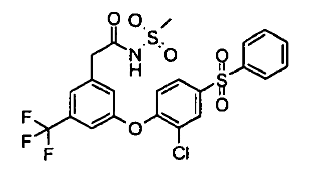

### (i) [3-[2-chloro-4-(phenylsulfonyl)phenoxy]-5-(trifluoromethyl)phenyl]acetic acid

The subtitle compound was prepared by the method of example reference 14 step (vi) using the product of example 5 step (i) and the product of reference example 16 step (i). 1H NMR DMSO-d6: δ 8.17 (1H, s), 8.01-7.99 (2H, d), 7.91-7.88 (1H, d), 7.72-7.61 (3H, m) 7.50 (1H, s) 7.37-7.31 (2H, d), 7.12-7.09 (1 H, d), 3.57 (2H, s).
MS: ESI(-ve) 425 (M-CO2).

### (ii) 2-[3-[2-chloro-4-(phenylsulfonyl)phenoxy]-5-(trifluoromethyl)phenyl]-N-(methylsulfonyl)acetamide

The title compound was prepared by the method of example 11 step (vi) using the product from step (i).
1H NMR DMSO-d6: δ 8.18 (1H, d), 8.03-8.01 (2H, d), 7.93-7.91 (1H, d), 7.74-7.34 (6H, m), 7.17-7.15 (1H, d), 3.76 (2H, s), 3.20 (3H, s).
MS: MM(-ve): 546 (M-H).

### Example 11

### 2-[3-[2-chtoro-4-(phenylsulfonyl)phenoxy]-5-(trifluoromethyl)phenyl]-N-(ethylsulfonyl)acetamide

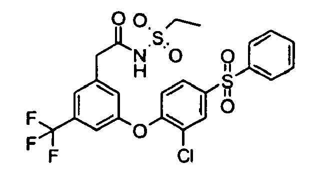

The title compound was prepared by the method of example 3 step (vi) using the product from example 10 step (i) and ethanesulfonamide.
1H NMR DMSO-d6: δ 8.19 (1H, s), 8.02-8.00 (2H, d), 7.91-7.88 (1H, d), 7.73-7.62 (3H, m), 7.50-7.30 (3H, m), 7.15-7.13 (1H, d), 3.42 (2H, s), 2.92-2.86 (2H, q), 0.98-0.95 (3H, t). MS: MM(-ve): 560 (M-H)

### Example 12

### 2-[3-[2-chloro-4-(ethylsulfonyl)phenoxy]-5-(trifluoromethyl)phenyl]-N-(ethylsulfonyl)propanamide

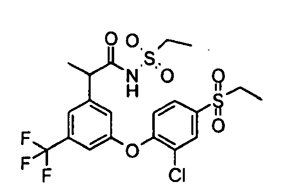

### (i) methyl [3-methoxy-5-(trifluoromethyl)phenyl]acetate

The product from example reference 13 step (iv) (1.0 g) was dissolved in dry DMF (10 ml) and treated with iodomethane (0.6 ml) and potassium carbonate (1.25 g). The mixture was stirred at RT overnight. The mixture was diluted with water, extracted with ether, dried (MgSO₄) and evaporated under reduced pressure to give an oil. The oil was purified by flash column chromatography (eluent 2:1 isohexane / ether) (1.30 g).
1H NMR DMSO-d6: 7.13 (1H, s), 7.04-7.01 (2H, d), 3.85 (3H, s), 3.70 (3H, s), 3.65 (2H, s)

### (ii) methyl 2-[3-methoxy-5-(trifluoromethyl)phenyl]propanoate

The product from step (i) (1.3 g) was added to a pre-formed solution of 2.5M butyllithium in hexanes (2.51 ml) and diisopropylamine (0.88 ml) in dry THF (30 ml) at -78 °C. The mixture was kept at -78 °C for 1 h before adding iodomethane (0.4 ml). The mixture was slowly allowed to warm to RT overnight. The mixture was diluted with 2M HCl, extracted with ether, dried (MgSO₄) and evaporated under reduced pressure to give an oil..The oil was purified by flash column chromatography (eluent 2:1 isohexane / ether) (0.8g).
1H NMR DMSO-d6: δ 7.17-7.13 (1H, s), 7.02 (2H, s), 3.84 (3H, s), 3.74 (1 H, q), 3.68 (3H, s), 1.52-1.50 (3H, d).

### (iii) 2-[3-hydroxy-5-(trifluoromethyl)phenyl]propanoic acid

The product from step (ii) (0.8 g) was dissolved in glacial acetic acid (20 ml) and treated with 48% aqueous HBr (20ml)). The mixture was heated at 100 °C for 16 h. The mixture was cooled and diluted with 2M NaOH, extracted with ethyl acetate, dried (MgSO₄) and evaporated under reduced pressure to give an oil, which was purified by RPHPLC to give a colourless oil (0.5 g).
MS: APCI(-ve): 233 (M-H).

### (iv) 2-[3-[2-chloro-4-(ethylsulfonyl)phenoxy]-5-(trifluoromethyl)phenyl]propanoic acid

The subtitle compound was synthesised by the method of reference example 14 step (vi) using the product of step (iii) and 2-chloro-4-(ethylsulfonyl)-1-fluorobenzene to give a white solid.
1H NMR DMSO-d6: δ 8.20-8.19 (1H, s), 8.14-8.11 (1H, d), 7.60 (1H, s), 7.50-7.47 (2H, d), 7.26-7.21 (1H, d), 3.85-3.80 (1H, q), 3.43-3.38 (2H, q), 1.39-1.38 (3H, d), 1.15-1.11 (3H, t).
MS: ESI(-ve) 425 (M-CO₂).

### (v) 2-[3-[2-chloro-4-(ethylsulfonyl)phenoxy]-5-(trifluoromethyl)phenyl]-N-(ethylsulfonyl)propanamide

The title compound was prepared by the method of example 11 step (vi) using the product from step (iv) and ethanesulfonamide.
1H NMR DMSO-d6: δ 7.86-7.83 (1H, d), 7.54-7.36 (4H, m), 7.25-7.23 (1H, d), 3.88-3.83 (1H, q), 3.41-3.23 (4H, m), 1.39-1.38 (3H, d), 1.17-1.04 (6H, m).
MS: MM(-ve): 526 (M-H).

### Example 13

### 2-[3-{[2-chloro-4-(ethylsulfonyl)phenyl]thiol}-5-(trifluoromethyl)phenyl]-N-(ethylsulfonyl)acetamide

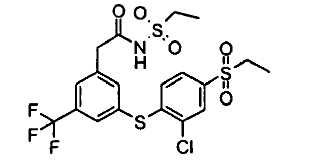

### i) [3-{[(dimethylamino)carbonothioyl]oxy}-5-(trifluoromethyl)phenyl]acetic acid

The product from example 5 step (i) (0.5 g), thiocarbamoyl chloride (0.32 ml), DMAP (0.026 g), triethylamine (0.60 ml) in dry dioxane (10 ml) was stirred at 100 °C for 15 h. The mixture was diluted with water, extracted with ethyl acetate, dried (MgSO₄) and evaporated under reduced pressure to an oil. The oil was purified by flash column chromatography (eluent ether) to give the subtitle compound (0.516 g).
¹H NMR CDCl₃: δ□ 7.42 (1H, s), 7.26-7.23 (2H, m), 3.72 (5H, m), 3.44 (3H, s), 3.35 (3H, s).

### ii) [3-{[(dimethylamino)carbonyl]thio}-5-(trifluoromethyl)phenyl]acetic acid

The product from step (i) (0.51 g) in diphenylether (5 ml) was heated at 200 °C for 20 h. The reaction mixture was purified by flash column chromatography (eluent: DCM to ether ) to give the subtitle compound (0.49 g).
MS: APCI(+ve) 322 (M+H).

### iii) [3-mercapto-5-(trifluoromethyl)phenyl]acetic acid

The product from step (ii) (0.49 g) was dissolved in methanol (10 ml) and 2M NaOH (10 ml) and stirred at RT overnight. The mixture was diluted with 2M HCl, extracted with ethyl acetate, dried (MgSO₄) and evaporated under reduced pressure.to an solid (0.30 g). ¹H NMR CDCl₃: δ□ 7.45 (1H, s), 7.32 (1H, s), 7.27-7.26 (1H, s), 3.76-3.66 (2H, s), 2.91 (1H, s).

### iv) [3-{[2-chloro-4-(ethylsulfonyl)phenyl]thio}-5-(trifluoromethyl)phenyl]acetic acid

The subtitle compound was synthesised by the method of reference example 14 step (vi) using the product of step (iii) and 2-chloro-4-(ethylsulfonyl)-1-fluorobenzene to give a white solid.
¹H NMR DMSO-d6: 7.97 (1H, s), 7.81-7.79 (3H, d), 7.73-7.69 (1H, d), 7.01-6.94 (1H, d), 3.63 (2H, s), 3.41-3.30 (2H, q), 1.19-1.06 (3H, t).
MS: APCI(-ve) 393 (M-CO₂).

### v) 2-[3-{[2-chloro-4-(ethylsulfonyl)phenyl]thio}-5-(trifluoromethyl)phenyl]-N-(ethylsulfonyl)acetamide

The title compound was prepared by the method of example 3 step (vi) using the product from step (iv) and ethanesulfonamide.
1H NMR DMSO-d6: δ 7.99-7.71 (5H, m), 7.03-6.99 (1H, d), 3.81 (2H, s), 3.37-3.26 (4H, m), 1.27-1.07 (6H, m).
MS: MM(-ve): 528 (M-H).

### Example 14

### 2-[3-{[2-chloro-4-(ethylsulfonyl)phenyl]thio}-5-(trifluoromethyl)phenyl]-N-(methylsulfonyl)acetamide

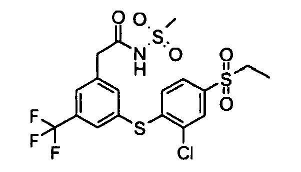

The title compound was prepared by the method of example 3 step (vi) using the product from example 39 step (iv) and methanesulfonamide.
1H NMR DMSO-d6: δ 8.01-7.70 (5H, m), 7.04-7.00 (1H, d), 3.78 (2H, s), 3.37-3.31 (2H, q), 3.09 (3H, s), 1.11-1.07 (3H, t).
MS: MM(-ve): 513 (M-H).

### Example 15

### N-(tert-butylsulfonyl)-2-[3-[2-cyano-4-(phenylsulfonyl)phenoxy]-5-(trifluoromethyl) phenyl]acetamide

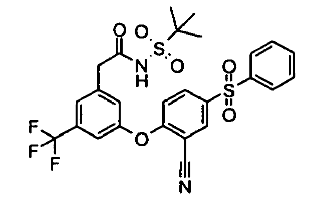

The title compound was prepared by the method of example 3 step (vi) using the product from example 7 step (i) and tert-butylsulfonamide.
1H NMR DMSO-d6: δ 8.62 (1H, d), 8.18 (1h, dd), 8.05-8.01 (2H, m), 7.75-7.70 (1H, m), 7.69-7.62 (4H, m), 7.51 (1H, s), 7.04 (1H, d), 3.77 (2H, s), 1.26 (9H, s).
MS: MM(-ve): 579 (M-H).

### Example 16

### 2-[3-[2-cyano-4-(phenylsulfonyl)phenoxy]-5-(trifluoromethyl)pheny]-N-(isopropyl sulfonyl)acetamide

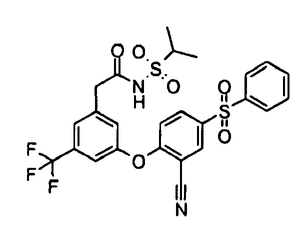

The title compound was prepared by the method of example 3 step (vi) using the product from example 7 step (i) and isopropylsulfonamide.
1H NMR DMSO-d6: δ 8.62 (1H, d), 8.61 (1H, d), 8.17 (1H, dd), 8.04-8.00 (2H, m), 7.75-7.70 (1H, m), 7.67-7.61 (4H, m), 7.49 (1H, s), 7.05 (1H, d), 3.62 (2H, s), 3.48-3.40 (1H, m), 1.12 (6H, d).
MS: MM(-ve): 565 (M-H).

### Example 17

### 2-[3-[2-chloro-4-(methylsulfonyl)phenoxy]-5-(trifluoromethyl)phenyl]-N-[(2,2,2-trifluoroethyl)sulfonyl]acetamide

The title compound was prepared by the method of example 3 step (vi) using the product from example 6 step (i) and 2,2,2-trifluoroethanesulfonamide.
1H NMR DMSO-d6: δ 8.18 (1H, d), 7.90 (1H, dd), 7.54 (1H, s), 7.47 (1H, s), 7.34 (1H, s), 7.26 (1H, d), 4.74 (2H, q), 3.83 (2H, s), 3.29 (3H, s).
MS: MM(-ve): 552 (M-H).

### Example 18

### 2-(4-chloro-2-{[2-chloro-4-(methylsulfonyl)phenyl]amino}lphenoxy)-N-(methylsulfonyl) acetamide

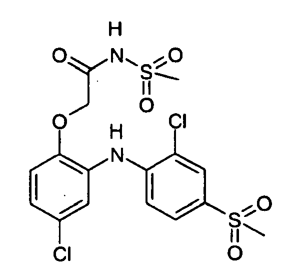

The title compound was prepared by the method of example 3 step (vi) using (4-chloro-2-{[2-chloro-4-(methylsulfonyl)phenyl]amino}phenoxy)acetic acid (WO2005018529) and methanesulfonamide.
1H NMR DMSO-d6: δ 11.99 (1H, s), 7.95 (1H, s), 7.88 (1H, d), 7.64 (1H, dd), 7.36 (1H, d), 7.23 (1H, dd), 7.05 (1H, d), 6.99 (1H, d), 4.77 (2H, s), 3.24 (3H, s), 3.18 (3H, s). MS: MM(+ve): 467 (M+H).

### Example 19

### 2-[3-[2-chloro-4-(methylsulfonyl)phenoxy]-5-(trifluoromethyl)phenyl]-N-(ethylsulfonyl) acetamide

The title compound was prepared by the method of example 3 step (vi) using the product from example 6 step (i) and ethanesulfonamide.
1H NMR DMSO-d6: δ 8.16 (1H, s), 7.90-7.87 (1H, d), 7.53-7.25 (4H, m), 3.64 (2H, s), 3.29 (3H, s), 3.18-3.12 (2H, q), 1.12-1.08 (3H, t).
MS: MM(-ve): 498 (M-H).

### Example 20

### 2-[3-[2-chloro-4-(methylsulfonyl)phenoxy]-5-(trifluoromethyl)phenyl]-N-(propylsulfonyl)acetamide

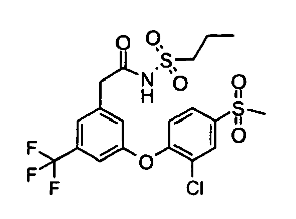

The title compound was prepared by the method of example 3 step (vi) using the product from example 6 step (i) and n-propylsulfonamide.
1H NMR DMSO-d6: δ 8.15-8.14 (1H, s), 7.88-7.86 (1H, d), 7.51 (1H, s), 7.41 (1H, s), 7.31 (1H, s), 7.25-7.23 (1H, d), 3.70 (2H, s), 3.27-3.09 (5H, m), 1.64-1.55 (2H, m), 0.91-0.88 (3H, t).
MS: MM(+ve): 514 (M+H).

### Example 21

### N-(benzylsulfonyl)-2-[3-[2-chloro-4-(methylsulfonyl)phenoxy]-5-(trifluoromethyl)phenyl]acetamide

The title compound was prepared by the method of example 3 step (vi) using the product from example 6 step (i) and 1-phenylmethanesulfonamide.
1H NMR DMSO-d6: δ 8.17 (1H, s), 7.91-7.88 (1H, d), 7.52-7.37 (2H, d), 7.34-7.18 (7H, m), 4.57 (2H, s), 3.61 (2H, s), 3.32 (3H, s).
MS: MM(+ve): 560 (M+H).

### Example 22

### N-(ethylsulfonyl)-2-[3-[4-(methylsulfonyl)-2-(trifluoromethyl)phenoxy]-5-(trifluoromethyl)phenyl]acetamide

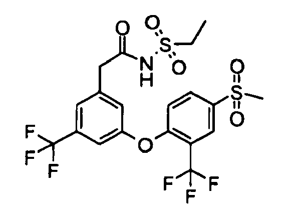

### (i) [3-[4-(methylsulfonyl)-2-(trifluoromethyl)phenoxy]-5-(trifluoromethyl)phenyl]acetic acid

The subtitle compound was prepared by the method of reference example 14 step (vi) using the product of example 5 step (i) and 1-fluoro-4-(methylsulfonyl)-2-(trifluoromethyl)benzene. The product was used as crude without further purification or characterisation.

### (ii) N-(ethylsulfonyl)-2-[3-[4-(methylsulfonyl)-2-(trifluoromethyl)phenoxy]-5-(trifluoromethyl)phenyl]acetamide

The title compound was prepared by the method of example 11 step (vi) using the product from step (i) and ethanesulfonamide.
1H NMR DMSO-d6: δ 8.27 (1H, s), 8.20-8.18 (1H, d), 7.60-7.43 (3H, m), 7.27-7.24 (1H, d), 3.69 (2H, s), 3.31 (3H, s), 3.22-3.16 (2H, q), 1.13-1.06 (3H, t).
MS: MM(-ve): 534 (M-H).

### Example 23

### 2-[3-[4-(methylsulfonyl)-2-(trifluoromethyl)phenoxy]-5-(trifluoromethyl)phenyl]-N-(propylsulfonyl)acetamide

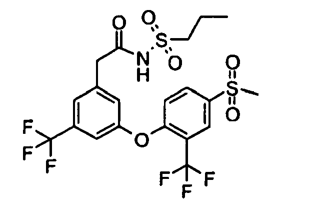

The title compound was prepared by the method of example 3 step (vi) using the product from example 22 step (i) and n-propylsulfonamide.
1H NMR DMSO-d6: δ 8.25 (1H, s), 8.18-8.15 (1H, d), 7.59-7.42 (3H, m), 7.24-7.22 (1H, d), 3.77 (2H, s), 3.29-3.25 (5H, m), 1.66-1.57 (2H, q), 0.92-0.89 (3H, t).
MS: MM(-ve): 546 (M-H).

### Example 24

### 2-{5-[2-chloro-4-(ethylsulfonyl)phenoxy]biphenyl-3-yl}-N-(ethylsulfonyl)acetamide

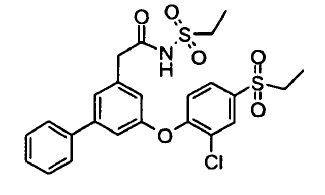

### (i) 3-bromo-5-methoxybenzonitrile

Sodium methoxide (2.02 g) was added to a stirred solution of 3-fluoro-5-bromobenzonitile (5.0 g) in DMPU (20 ml) and stirred at RT for 2 h. The reaction was diluted with water and the resulting solid formed was filtered and washed with water, then dried in vacuo to give the subtitle compound (5.10g).
1H NMR DMSO-d6: δ□ 7.39-7.38 (1H, s), 7.30-7.26 (1H, m), 7.11 (1H, s), 3.83 (3H, s).

### (ii) 3-bromo-5-methoxybenzoic acid

The product from step (i) (5.10g) was dissolved in methanol (20ml) and 6N NaOH (20ml) and heated to reflux for 6 h. The mixture was diluted with 2M HCl, extracted with ethyl acetate, dried (MgSO₄) and evaporated under reduced pressure to give a white solid (5.10g).
MS: APCI(-ve) 229 (M-H).

### (iii) (3-bromo-5-methoxyphenyl)methanol

Lithium aluminium hydride (1M in THF, 22.07 ml) was added dropwise to a stirred solution of the product of step (ii) (5.1 g) in THF (50 ml) at 0 °C and stirred at RT overnight. The reaction was quenched in 2M HCl, extracted with ether, dried (MgSO₄) and evaporated under reduced pressure to give an oil, which was purified by flash column chromatography (eluent 1:1 isohexane/ether) to give the subtitle compound (5.38 g).
1H NMR CDCl3: δ 7.08 (1H, s), 6.96-6.91 (1H, s), 6.83-6.81 (1H, s), 4.62 (2H, s), 3.79 (3H, s).

### (iv) (3-bromo-5-methoxyphenyl)acetonitrile

The product from step (iii) (5.38 g) was dissolved in dry DCM (50 ml) and dry DMF (2.3 ml) added followed by thionyl chloride (2.17 ml). The reaction mixture was stirred at RT overnight, and then diluted with aqueous sodium hydrogen carbonate, extracted with DCM, dried (MgSO₄) and evaporated under reduced pressure to give an oil. The oil was dissolved in DMF (20 ml), sodium cyanide (1.30 g) was added and stirred at RT overnight. The reaction mixture was diluted with water, extracted with ether, dried (MgSO₄) and evaporated under reduced pressure. The residue was purified by flash column chromatography (eluent 1:2 ether isohexane) to give the subtitle compound (4.50 g).
1H NMR CDCl3 δ□ 7.07-7.02 (2H, m), 6.81 (1H, s), 3.83 (3H, s), 3.70 (2H, s).

### (v) (3-bromo-5-hydroxyphenyl)acetic acid

The product of step (iv) (4.5 g), in glacial acetic acid (30 ml) was treated with 48 % aqueous HBr (30 ml) and heated at 100 °C for 24 h. The reaction mixture was partitioned between water and ethyl acetate, the organics were separated then dried (MgSO₄) and evaporated under reduced pressure to give a tan solid which was triturated with ether/isohexane (4.24g).
MS: APCI(-ve) 229/231 (M-H).

### (vi) {3-bromo-5-[2-chloro-4-(ethylsulfonyl)phenoxy]phenyl}acetic acid

The subtitle compound was prepared as described in reference example 14 step (vi) but instead using the product from step (v) and 2-chloro-4-(ethylsulfonyl)-1-fluorobenzene. 1H NMR DMSO-d6: 8.08-8.07 (1H, s), 7.84-7.81 (1H, d), 7.38-7.20 (3H, m), 7.07 (1H, s), 3.59 (2H, s), 3.39-3.34 (2H, q), 1.14-1.07 (3H, t).
MS: APCI(-ve) 431/433 (M-H).

### (vii) {5-[2-chloro-4-(ethylsulfonyl)phenoxy]biphenyl-3-yl}acetic acid

The product from step (vi) (0.45 g), phenyl boronic acid (0.190 g), sodium carbonate (0.44 g) and bisdiphenylphosphinoferrocene palladium (II) (0.04 g) in dry dioxane (20 ml) were heated to 80 °C for 20 h. The mixture was diluted with 2M HCl, extracted with ethyl acetate, dried (MgSO₄) and evaporated under reduced pressure to an oil, which was purified by RPHPLC to give a cream solid.
1H NMR DMSO-d6: δ 8.07 (1H, s), 7.82-7.79 (1H, d), 7.69-7.66 (2H, d), 7.50-7.35 (5H, m), 7.18-7.06 (2H, m), 3.69 (2H, s), 3.39-3.31 (2H, q), 1.14-1.09 (3H, t).
MS: MM-ve 385 (M-CO₂).

### (viii) 2-{5-[2-chloro-4-(ethylsulfonyl)phenoxy]biphenyl-3-yl}-N-(ethylsulfonyl)acetamide

The product from step (vii) (0.27 g) in dry THF (10 ml) was treated with CDI (0.10 g) and heated at 60 °C for 1 h. Mixture was allowed to cool to RT and ethanesulfonamide (0.07 g) was added and the mixture stirred at RT for 10 mins before adding DBU (0.09 ml). After 1 h the mixture was diluted with 2M HCl, extracted with ethyl acetate and the organics dried (MgSO₄) and evaporated under reduced pressure to an oil. The oil was dissolved in a solution of methanol (20 ml) and acetyl chloride (5 ml) and stirred at RT for 1 h then evaporated under reduced pressure to an oil. The oil was purified by RPHPLC to give a white solid (0.02 g). 1H NMR DMSO-d6: δ 8.08-8.06 (1H, m), 7.83-7.37 (8H, m), 7.19-7.12 (1H, d), 6.95 (1H, s), 3.70 (2H, s), 3.38-3.26 (4H, m), 1.16-1.08 (6H, m).
MS: MM(+ve): 522 (M+H).

### Example 25

### 2-{3-chloro-5-[4-(methylsulfonyl)-2-(trifluoromethyl)phenoxyl]phenyl}-N-[(trifluoromethyl)sulfonyl]acetamide

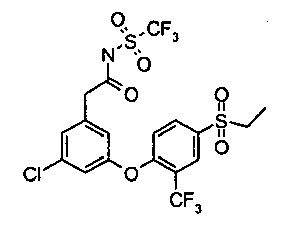

### (i) {3-chloro-5-[4-(methylsulfonyl)-2-(trifluoromethyl)phenoxy]phenyl}acetic acid

The subtitled compound was prepared by the method of example 3 step (v) using the product of example 3 step (iv) and 1-fluoro-4-(methylsulfonyl)-2-(trifluoromethyl)benzene.1H NMR DMSO-d6: δ 8.24-8.15 (2H, m), 7.27-7.16 (3H, m), 7.05 (1H, s), 3.54 (2H, s), 3.46 (2H, q), 1.15-1.06 (3H, t).
MS: ESI-ve 377 (M-CO₂).

### (ii) 2-{3-chloro-5-[4-(methylsulfonyl)-2-(trifluoromethyl)phenoxy]phenyl}-N-[(trifluoromethyl)sulfonyl]acetamide

A solution of the product from step (i) (40 mg) and 1,1,1-trifluoromethanesulfonamide (42 mg) in DCM (2 ml) were treated with Hunigs base then HATU and the mixture stirred overnight at RT. The mixture was diluted with 1M HCl and the DCM layer passed directly onto a Varian NH₂ cartridge (eluted with MeCN then 20% TFA in MeCN). The acidic fraction was concentrated and the residue purified by RPHPLC to give the title compound as a white solid (22 mg).
1H NMR DMSO-d6: δ 8.17 (1H, d), 8.11 (1H, dd), 7.27 (1H, s), 7.14-7.21 (3H, m), 3.47 (2H, s), 3.40 (2H, q), 1.13 (3H, t).
MS: MM-ve 377 (M-H).

### Example 26

### 2-{3-chloro-5-[2-chloro-4-(4-fluorobenzoyl)phenoxy]phenyl}-N-[(2,2,2-trifluoroethyl)sulfonyl]acetamide

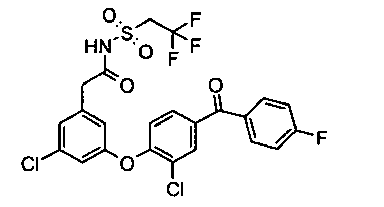

### (i) (3-chloro-4-fluorophenyl)(4-fluorophenyl)methanone

The subtitle compound was prepared by the method of reference example 20 step (i) using 4-fluorobenzene.
1H NMR CDCI3-d6: δ 7.88-7.78 (3H, m), 7.57-7.53 (1H, m), 7.29-7.15 (3H, m).

### (ii) {3-chloro-5-[2-chloro-4-(4-fluorobenzoyl)phenoxy]phenyl}acetic acid

The title compound was prepared as described in reference example 2 step (iii) but instead using the product from reference example 14 step (vi) and the product from step (i).
1H NMR DMSO-d6: δ 7.92-7.84 (3H, m), 7.72-7.69 (1H, d), 7.44-7.39 (2H, m), 7.21-7.17 (2H, m), 7.10-7.08 (1H, m), 7.00 (1H, s), 3.55 (2H, s).
MS: APCI+ve 373 (M-CO₂).

### (iii) 2-{3-chloro-5-[2-chloro-4-(4-fluorobenzoyl)phenoxy]phenyl}-N-[(2,2,2-trifluoroethyl)sulfonyl]acetamide

The title compound was prepared by the method of example 25 step (ii) using the product from step (ii) and 2,2,2-trifluoroethanesulfonamide. Purification by RPHPLC gave a white solid (32 mg).
1H NMR DMSO-d6: δ 7.93 (1H, d), 7.89-7.85 (2H, m), 7.71 (1H, dd), 7.41 (2H, t), 7.22-7.20 (2H, m), 7.16 (1H, t), 7.00 (1H, s), 4.65-4.58 (2H, m), 3.66 (2H, s)
MS: MM-ve 562 (M-H).

### Example 27

### 2-[3-[2-cyano-4-(phenylsulfonyl)phenoxy]-5-(trifluoromethyl)phenyl]-N-(propylsulfonyl)acetamide

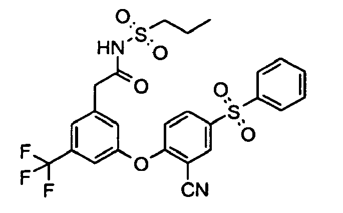

The title compound was prepared by the method of example 25 step (ii) using the product from example 7 step (i) and *n*-propylsulfonamide.
1H NMR DMSO-d6: δ 8.60 (1H, s), 8.19-8.13 (1H, m), 8.03-7.98 (2H, m), 7.50 (1H, s), 7.03 (1H, dd), 7.72-7.60 (5H, m), 3.78 (2H, s), 3.32-3.24 (2H, m), 1.65-1.58 (2H, m), 0.93-0.88 (3H, m).
MS: MM(-ve): 565 (M-H).

### Example 28

### 2-{3-[2-chloro-4-(ethylsutfonyl)phenoxy]-5-fluorophenyl}-N-(propylsulfonyl)acetamide

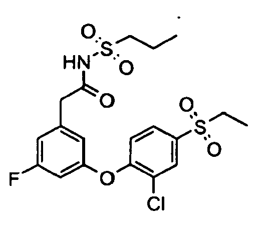

### (i) 3-fluoro-5-methoxybenzoic acid

The subtitle compound was prepared by the method of example 3 step (i) using 3,5-difluoro-benzoic acid.
MS: APCI-ve 169 (M-H).

### (ii) (3-fluoro-5-methoxyphenyl)methanol

The subtitle compound was prepared by the method of example 3 step (ii) using the product of step (i).
¹H NMR CDCl₃-d6: δ 6.68 (2H, m), 6.53 (1H, m), 4.67 (2H, d), 3.8 (3H, s).

### (iii) (3-fluoro-5-methoxyphenyl)acetonitrile

Thionyl chloride (0.95 ml) was added to a solution of the product of step (ii) (0.95 ml) in DCM (20 ml) at 0 °C, then stirred for 1 h. The reaction mixture was washed with 2M HCl, the organics were separated then dried (MgSO₄) and evaporated under reduced pressure. The residue was dissolved in DMF (10 ml) and sodium cyanide (0.3 g) was added. The mixture was stirred for 2 h, then partitioned between ether and water; the organics were separated, then dried (MgSO₄) and evaporated under reduced pressure. The residue was purified by flash column chromatography (eluent 1:1 ether/ isohexane) to give the subtitle compound (0.47 g).
¹H NMR CDCl₃-d6: δ 6.66 (3H, m), 3.81 (3H, s), 3.70 (2H, s).

### (iv) (3-fluoro-5-hydroxyphenyl)acetic acid

The subtitle compound was prepared by the method of example 3 step (iv) using the product of step (iii).
¹H NMR DMSO-d6: δ 12.10 (1H, s), 9.79 (1H, s), 6.42 (3H, m), 3.44 (2H, s).

### (v) {3-[2-chloro-4-(ethylsulfonyl)phenoxy]-5-fluorophenyl}acetic acid

The subtitle compound was prepared by the method of example 3 step (v) using the product of step (iv) and -chloro-4-(ethylsulfonyl)-1-fluorobenzene.
¹H NMR DMSO-d6: δ 8.07 (1H, m), 7.82 (1H, d), 7.22 (1H, d), 7.03-6.89 (3H, m), 3.58 (2H, s), 3.32 (2H, q), 1.12 (3H, t).
MS: APCI-ve 371 (M-H).

### (vi) 2-{3-[2-chloro4-(ethylsulfonyl)phenoxy]-5-fluorophenyl}-N-(propylsulfonyl) acetamide

The title compound was prepared by the method of example 25 step (ii) using the product from step (v) and *n*-propylsulfonamide.
1H NMR DMSO-d6: δ 11.86 (1H, s), 8.09 (1H, d), 7.84 (1H, dd), 7.25 (1H, d), 7.07-7.00 (2H, m), 6.91 (1H, s), 3.67 (2H, s), 3.37 (2H, q), 3.31 (2H, m), 1.64 (2H, sextet), 1.13 (3H, t), 0.94 (3H, t).
MS: MM(-ve): 478 (M-H).

### Example 29

### 2-{3-chloro-5-[2-chloro-4-(ethylsulfonyl)phenoxy]phenyl}-N-(propylsulfonyl)acetamide

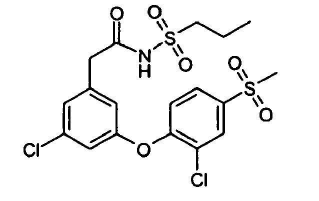

The title compound was prepared by the method of example 25 step (ii) using the product from example 3 step (v) and *n*-propylsulfonamide.
1H NMR DMSO-d6: δ 11.86 (1H, s), 8.16-8.15 (1H, s), 7.91-7.87 (1H, d), 7.27-7.19 (3H, m), 7.02-7.01 (1H, m), 3.68 (2H, s), 3.35-3.32 (2H, m), 3.28 (3H, s), 1.71-1.58 (2H, q), 0.97-0.92 (3H, t).
MS: MM(-ve): 478 (M-H).

### Example 30

### 2-{3-chloro-5-(2-chloro-4-(ethylsulfonyl)phenoxy]phenyl}-N-(ethylsulfonyl)acetamide

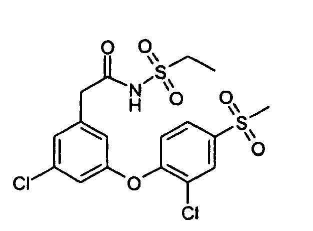

The title compound was prepared by the method of example 25 step (ii) using the product from example 3 step (v) and n-ethylsulfonamide.
1H NMR DMSO-d6: δ 8.16 (1H, d), 7.89 (1H, dd), 7.27-7.25 (2H, m), 7.19 (1 H, t), 7.02 (1H, t), 3.66 (2H, s), 3.26-3.25 (5H, m), 1.16 (3H, t).
MS: MM(-ve): 464 (M-H).

### Example 31

### 2-{3-chloro-5-[2-chloro-4-(ethylsulfonyl)phenoxy]phenyl}-N-(isobutylsulfonyl)acetamide

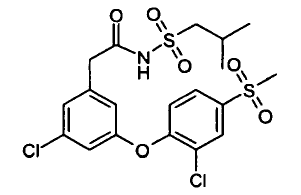

The title compound was prepared by the method of example 25 step (ii) using the product from example 3 step (v) and isobutylsulfonamide.
1H NMR DMSO-d6: δ 11.94 (1H, s), 8.18-8.17 (1H, s), 7.91-7.88 (1H, d), 7.54 (1H, s), 7.46 (1H, s), 7.33-7.26 (2H, m), 3.78 (2H, s), 3.29-3.25 (5H, m), 2.08-2.01 (1H, m), 0.97-0.96 (6H, d).
MS: MM(-ve): 526 (M-H).

### Pharmacological Data

### Ligand Binding Assay

[³H]PGD₂ was purchased from Perkin Elmer Life Sciences with a specific activity of 100-210Ci/mmol. All other chemicals were of analytical grade.

HEK cells expressing rhCRTh2 / Gα16 were routinely maintained in DMEM containing 10% Foetal Bovine Serum (HyClone), 1mg/ml geneticin, 2mM L-glutamine and 1% non-essential amino acids. For the preparation of membranes, the adherent transfected HEKcells were grown to confluence in two layer tissue culture factories (Fisher, catalogue number TKT-170-070E). Maximal levels of receptor expression were induced by addition of 500mM sodium butyrate for the last 18 hours of culture. The adherent cells were washed once with phosphate buffered saline (PBS, 50ml per cell factory) and detached by the addition of 50ml per cell factory of ice-cold membrane homogenisation buffer [20mM HEPES (pH 7.4), 0.1mM dithiothreitol, 1mM EDTA, 0.1mM phenyl methyl sulphonyl fluoride and 100µg/ml bacitracin]. Cells were pelleted by centrifugation at 220xg for 10 minutes at 4°C, re-suspended in half the original volume of fresh membrane homogenisation buffer and disrupted using a Polytron homogeniser for 2 x 20 second bursts keeping the tube in ice at all times. Unbroken cells were removed by centrifugation at 220xg for 10 minutes at 4°C and the membrane fraction pelleted by centrifugation at 90000xg for 30 minutes at 4°C. The final pellet was re-suspended in 4 ml of membrane homogenisation buffer per cell factory used and the protein content determined. Membranes were stored at -80°C in suitable aliquots.

All assays were performed in Coming clear bottomed, white 96-well NBS plates (Fisher). Prior to assay, the HEK cells membranes containing CRTh2 were coated onto SPA PVT WGA beads (Amersham). For coating membranes were incubated with beads at typically 25µg membrane protein per mg beads at 4°C with constant agitation overnight. (The optimum coating concentrations were determined for each batch of membranes) The beads were pelleted by centrifugation (800xg for 7minutes at 4°C), washed once with assay buffer (50mM HEPES pH 7.4 containing 5mM magnesium chloride) and finally re-suspended in assay buffer at a bead concentration of 10mg/ml.

Each assay contained 20µl of 6.25nM [³H]PGD₂, 20µl membrane saturated SPA beads both in assay buffer and 10µl of compound solution or 13,14-dihydro-15-keto prostaglandin D₂ (DK-PGD₂, for determination of non-specific binding, Cayman chemical company).

Compounds and DK-PGD₂ were dissolved in DMSO and diluted in the same solvent to 100x the required final concentration. Assay buffer was added to give a final concentration of 10% DMSO (compounds were now at 10x the required final concentration) and this was the solution added to the assay plate. The assay plate was incubated at RT for 2 hours and counted on a Wallac Microbeta liquid scintillation counter (1 minute per well).

Compounds of formula (I) have an IC₅₀ value of less than (<) 10µM. Specifically example 2 has a pIC₅₀ value of 6.85.

## Claims

1. A compound of formula (I) or a pharmaceutically acceptable salt thereof: 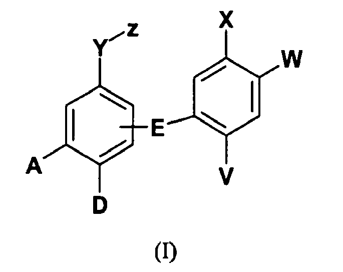 in which:
A and D are independently selected from hydrogen, halogen, phenyl or C₁₋₃ alkyl, the latter being optionally substituted by halogen atoms;
E is O, S, NR^{3'} or CH₂;
V is halogen, CN or C₁₋₃alkyl, the latter being optionally substituted by halogen atoms;
W is selected from SO₂R⁶ and COR⁴;
X is hydrogen or CF₃;
Y is selected from O-CR¹R², CR¹R² and CR¹R²CR¹R²;
Z is CONHSO₂R⁶;
R¹ and R² independently represent a hydrogen atom, halogen, C₃₋₈cycloalkyl or a C₁₋₆ alkyl group, the latter two groups being optionally substituted by one or more halogen atoms; or
R¹ and R² together can form a 3-8 membered ring optionally containing one or more atoms selected from O, S, NR³ and itself optionally substituted by one or more halogen, C₃₋₈ cycloalkyl or C₁-₃ alkyl the latter two groups being optionally substituted by one or more halogen or NR⁴R⁵;
R³ represents hydrogen, C₃₋₈ cycloalkyl or C₁-₆ alkyl the latter two groups being optionally substituted by halogen atoms;
R^{3'} represents hydrogen, C₃₋₈ cycloalkyl or C₁-₆ alkyl the latter two groups being optionally substituted by halogen atoms;
R⁴ and R⁵ independently represent hydrogen, C₃₋₈ cycloalkyl or C₁₋₆alkyl the latter two groups being optionally substituted by one or more substituents independently selected from halogen, CN, C₃-₇ cycloalkyl, C₁₋₆ alkyl, OR³ and NR⁷R⁸, aryl, heteoaryl, S(O)ₙR⁹ (where n = 0,1 or 2), CONR⁷R⁸, NR³COR¹⁰, SO₂NR⁴R⁵ and NR³SO₂R⁹; or
R⁴ and R⁵ together with the nitrogen atom to which they are attached can form a 3-8 membered saturated ring optionally containing one or more atoms selected from O, N, S(O)ₙ (where n = 0,1 or 2), NR³, and itself optionally substituted by one or more halogen, OR³, C₃₋₈ cycloalkyl or C₁₋₆ alkyl, the latter two groups being optionally substituted by one or more halogen;
R⁶ represents aryl, heteroaryl, C₃₋₈ cycloalkyl or C₁₋₆alkyl all of which being optionally substituted by one or more substituents independently selected from halogen, C₃-₇ cycloalkyl, C₁₋₆ alkyl, OR³, CN, NR⁷R⁸, aryl, heteoaryl, S(O)ₙR⁹ (where n = 0,1 or 2), CONR⁷R⁸, NR³COR¹⁰, SO₂NR⁴R⁵ and NR³SO₂R⁹;
R⁷ and R⁸ independently represent hydrogen, C₃₋₈ cycloalkyl or C₁₋₆alkyl, the latter two groups being optionally substituted by one or more halogen atoms; or
R⁷ and R⁸ together with the nitrogen atom to which they are attached can form a 3-8 membered saturated ring, optionally substituted by halogen, C₃₋₈ cycloalkyl or C₁₋₃ alkyl;
R⁹ represents C₃₋₈ cycloalkyl or C₁-₆ alkyl (optionally substituted by halogen atoms);
R¹⁰ represents hydrogen, aryl, heteroaryl, OR₃, C₃₋₈ cycloalkyl or C₁₋₆ alkyl the latter three groups being optionally substituted by halogen atoms;
R¹¹ and R¹² independently represent hydrogen, aryl, heteroaryl, C₃₋₈ cycloalkyl or C₁₋₈ alkyl, the latter being optionally substituted by one or more halogen atoms, OR³, =O or NR¹³R¹⁴; or
R¹¹ and R¹² together with the nitrogen atom to which they are attached can form a 3-8 membered ring optionally containing one or more atoms selected from O, S, NR³. This ring is itself optionally substituted by one or more C₃₋₈ cycloalkyl, C₁₋₃ alkyl, halogen, OR³ or NR¹³R¹⁴;
R¹³ and R¹⁴ independently represent hydrogen, C₃₋₈ cycloalkyl or C₁₋₆ alkyl the latter two groups being optionally substituted by one or more substituents independently selected from halogen, OR³, CN, S(O)ₙR⁹ (where n = 0,1 or 2), CONR⁷R⁸, NR³COR¹⁰, SO₂NR⁴R⁵ and NR³SO₂R⁹;
or
R¹³ and R¹⁴ together with the nitrogen atom to which they are attached can form a 5-10 membered unsaturated heteroaryl ring optionally containing one or more atoms selected from O, N, S(O)ₙ (where n = 0,1 or 2), NR³, and itself optionally substituted by halogen, CN, S(O)ₙR⁹ (where n = 0, 1 or 2), OR³, C₃₋₈ cycloalkyl or C₁₋₃ alkyl;
R¹⁵ represents one or more substituents independently selected from hydrogen, halogen, CN, OR³, S(O)ₙR⁹ (where n = 0, 1 or 2), C₃₋₈ cycloalkyl or C₁₋₆ alkyl the latter two groups being optionally substituted by halogen atoms,
provided that:
• A and D cannot both be hydrogen

2. A compound according to claim 1 in which E is O or S.

3. A compound according to claims 1 or 2 in which A and D are independently selected from hydrogen, halogen or C₁₋₃ alkyl, the latter being optionally substituted by halogen atoms.

4. A compound according to claims 1 or 2 in which A and D are independently selected from hydrogen, halogen or CF₃.

5. A compound according to claims 1 or 2 in which when A is hydrogen D is halogen or C₁₋₃ alkyl, the latter being optionally substituted by halogen atoms.

6. A compound according to claims 1 or 2 in which when D is hydrogen A is halogen, phenyl or C₁₋₃ alkyl, the latter being optionally substituted by halogen atoms.

7. A compound according to claims 1 or 2 in which when A is hydrogen D is halogen or CF₃; when D is hydrogen A is halogen or CF₃.

8. A compound according to any of claims 1 to 7 in which Y is CR¹R^{2.}

9. A compound according to claim 8 in which R¹ and R² are both hydrogen.

10. A compound according to any one of claims 1 to 9 in which W is SO₂R⁶.

11. A compound according to any one of claims 1 to 10 in which E is connected to the aromatic ring in the *ortho-* position with respect to Y.

12. A compound according to any one of claims 1 to 10 in which E is connected to the aromatic ring in the *meta-* position with respect to Y.

13. A compound of formula (I) as defined in any one of claims 1 to 12 selected from:
2-[2-[2-chloro-4-(methylsulfonyl)phenoxy]-4-(trifluoromethyl)phenoxy]-*N-*(methylsulfonyl)acetamide;
2-{4-chloro-3-[2-chloro-4-(methylsulfonyl)phenoxy]phenyl}-*N-*(methylsulfonyl) acetamide;
2-{3-chloro-5-[2-chloro-4-(methylsulfonyl)phenoxy]phenyl}-*N-*(methylsulfonyl) acetamide;
2-(3-chloro-5-{[2-fluoro-4-(phenylsulfonyl)phenyl]thio}phenyl)-N-(methylsulfonyl) acetamide;
2-[3-{2-chloro-4-[(4-fluorobenzyl)sulfonyl]phenoxy}-5-(trifluoromethyl)phenyl]-*N-*(methylsulfonyl)acetamide;
2-[3-[2-chloro-4-(methylsulfonyl)phenoxy]-5-(trifluoromethyl)phenyl]-*N-*(methylsulfonyl)acetamide;
2-[3-[2-cyano-4-(phenylsulfonyl)phenoxy]-5-(trifluoromethyl)phenyl]-*N-*(methylsulfonyl)acetamide;
2-[3-[4-(Ethylsulfonyl)-2-(trifluoromethyl)phenoxy]-5-(trifluoromethyl)phenyl]-*N-*(methylsulfonyl)acetamide;
2-[3-[2-cyano-4-(phenylsulfonyl)phenoxy]-5-(trifluoromethyl)phenyl]-*N-*(ethylsulfonyl)acetamide;
2-[3-[2-chloro-4-(phenylsulfonyl)phenoxy]-5-(trifluoromethyl)phenyl]-N-(methylsulfonyl)acetamide;
2-[3-[2-chloro-4-(phenylsulfonyl)phenoxy]-5-(trifluoromethyl)phenyl]-N-(ethylsulfonyl)acetamide;
2-[3-[2-chloro-4-(ethylsulfonyl)phenoxy]-5-(trifluoromethyl)phenyl]-N-(ethylsulfonyl)propanamide;
2-[3-{[2-chloro-4-(ethylsulfonyl)phenyl]thio}-5-(trifluoromethyl)phenyl]-N-(ethylsulfonyl)acetamide;
2-[3-{[2-chloro-4-(ethylsulfonyl)phenyl]thio}-5-(trifluoromethyl)phenyl]-N-(methylsulfonyl)acetamide;
N-(tert-butylsulfonyl)-2-[3-[2-cyano-4-(phenylsulfonyl)phenoxy]-5-(trifluoromethyl) phenyl] acetamide;
2-[3-[2-cyano-4-(phenylsulfonyl)phenoxy]-5-(trifluoromethyl)phenyl]-N-(isopropyl sulfonyl)acetamide;
2-[3-[2-chloro-4-(methylsulfonyl)phenoxy]-5-(trifluoromethyl)phenyl]-N-[(2,2,2-trifluoroethyl)sulfonyl]acetamide;
2-(4-chloro-2-{[2-chloro-4-(methylsulfonyl)phenyl]amino}phenoxy)-N-(methylsulfonyl) acetamide;
2-[3-[2-chloro-4-(methylsulfonyl)phenoxy]-5-(trifluoromethyl)phenyl]-N-(ethylsulfonyl) acetamide;
2-[3-[2-chloro-4-(methylsulfonyl)phenoxy]-5-(trifluoromethyl)phenyl]-N-(propylsulfonyl)acetamide;
N-(benzylsulfonyl)-2-[3-[2-chloro-4-(methylsulfonyl)phenoxy]-5-(trifluoromethyl)phenyl]acetamide;
N-(ethylsulfonyl)-2-[3-[4-(methylsulfonyl)-2-(trifluoromethyl)phenoxy]-5-(trifluoromethyl)phenyl]acetamide;
2-[3-[4-(methylsulfonyl)-2-(trifluoromethyl)phenoxy]-5-(trifluoromethyl)phenyl]-N-(propylsulfonyl)acetamide;
2-{5-[2-chloro-4-(ethylsulfonyl)phenoxy]biphenyl-3-yl}-N-(ethylsulfonyl)acetamide;
2-{3-chloro-5-[4-(methylsulfonyl)-2-(trifluoromethyl)phenoxy]phenyl}-*N-*[(trifluoromethyl)sulfonyl]acetamide;
2-{3-chloro-5-[2-chloro-4-(4-fluorobenzoyl)phenoxy]phenyl}-N-[(2,2,2-trifluoroethyl)sulfonyl]acetamide;
2-[3-[2-cyano-4-(phenylsulfonyl)phenoxy]-5-(trifluoromethyl)phenyl]-N-(propylsulfonyl)acetamide;
2-{3-[2-chloro-4-(ethylsulfonyl)phenoxy]-5-fluorophenyl}-N-(propylsulfonyl)acetamide;
2-{3-chloro-5-[2-chloro-4-(ethylsulfonyl)phenoxy]phenyl}-N-(propylsulfonyl)acetamide;
2-{3-chloro-5-[2-chloro-4-(ethylsulfonyl)phenoxy]phenyl}-1-N-(ethylsulfonyl)acetamide;
2-{3-chloro-5-[2-chloro-4-(ethylsulfonyl)phenoxy]phenyl}-*N-*(isobutylsulfonyl)acetamide;
and pharmaceutically acceptable salts thereof.

14. A pharmaceutical composition comprising a compound of formula (I) or a pharmaceutically acceptable salt thereof as defined in any one of claims 1 to 13 in association with a pharmaceutically acceptable adjuvant, diluent or carrier.

15. A compound of formula (I) or a pharmaceutically acceptable salt thereof as defined in any one of claims 1 to 13 for use as a medicament.

16. A compound of formula (I) or a pharmaceutically acceptable salt thereof as defined in any one of claims 1 to 13 for use in the treatment or prophylaxis of asthma or COPD.

## Patentansprüche

1. Verbindungen der Formel (I) und deren pharmazeutisch annehmbare Salze: 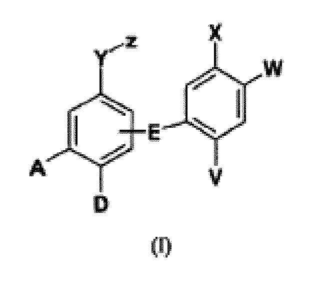 wobei:
A und D unabhängig voneinander aus Wasserstoff, Halogen, Phenyl und C₁₋₃-Alkyl ausgewählt sind,
wobei der letztgenannte Rest gegebenenfalls durch Halogenatome substituiert ist;
E für O, S, NR^{3'} oder CH₂ steht;
V für Halogen, CN oder C₁₋₃-Alkyl steht, wobei der letztgenannte Rest gegebenenfalls durch Halogenatome substituiert ist;
W aus SO₂R⁶ und COR⁴ ausgewählt ist;
X für Wasserstoff oder CF₃ steht;
Y aus O-CR¹R², CR¹R² und CR¹R²CR¹R² ausgewählt ist;
Z für CONHSO₂R⁶ steht;
R¹ und R² unabhängig voneinander für ein Wasserstoffatom, Halogen, C₃₋₈-Cycloalkyl oder eine C₁₋₆-Alkylgruppe stehen, wobei die beiden letztgenannten Gruppen gegebenenfalls durch ein oder mehrere Halogenatome substituiert sind; oder
R¹ und R² zusammen einen 3- bis 8-gliedrigen Ring bilden können, der gegebenenfalls ein oder mehrere aus O, S und NR³ ausgewählte Atome enthält und selbst gegebenenfalls durch ein oder mehrere Halogene, C₃₋₈-Cycloalkylreste oder C₁₋₃-Alkylreste substituiert ist, wobei die beiden letztgenannten Gruppen gegebenenfalls durch ein oder mehrere Halogene oder NR⁴R⁵ substituiert sind;
R³ für Wasserstoff, C₃₋₈-Cycloalkyl oder C₁₋₆-Alkyl steht, wobei die beiden letztgenannten Gruppen gegebenenfalls durch Halogenatome substituiert sind;
R^{3'} für Wasserstoff, C₃₋₈-Cycloalkyl oder C₁₋₆-Alkyl steht, wobei die beiden letztgenannten Gruppen gegebenenfalls durch Halogenatome substituiert sind;
R⁴ und R⁵ unabhängig voneinander für Wasserstoff, C₃₋₈-Cycloalkyl oder C₁₋₆-Alkyl stehen, wobei die beiden letztgenannten Gruppen gegebenenfalls durch einen oder mehrere Substituenten unabhängig voneinander ausgewählt aus Halogen, CN, C₃₋₇-Cycloalkyl, C₁₋₆-Alkyl, OR³ und NR⁷R⁸ Aryl, Heteroaryl, S(O)ₙR⁹ (wobei n = 0, 1 oder 2), CONR⁷R⁸ NR³COR¹⁰ SO₂NR₄R₅ und NR³SO₂R⁹ substituiert sind; oder
R⁴ und R⁵ zusammen mit dem Stickstoffatom, an das sie gebunden sind, einen 3- bis 8-gliedrigen gesättigten Ring bilden können, der gegebenenfalls ein oder mehrere Atome ausgewählt aus O, N, S(O)ₙ (wobei n = 0, 1 oder 2) und NR³ enthält und selbst gegebenenfalls durch ein oder mehrere Halogene, OR³, C₃₋₈-Cycloalkylreste oder C₁₋₆-Alkylreste substituiert ist, wobei die beiden letztgenannten Gruppen gegebenenfalls durch ein oder mehrere Halogene substituiert sind;
R⁶ für Aryl, Heteroaryl, C₃₋₈-Cycloalkyl oder C₁₋₆-Alkyl steht, welche alle gegebenenfalls durch einen oder mehrere Substituenten unabhängig voneinander ausgewählt aus Halogen, C₃₋₇-Cycloalkyl, C₁₋₆-Alkyl, OR³, CN, NR⁷R⁸ Aryl, Heteroaryl, S(O)ₙR⁹ (wobei n = 0, 1 oder 2), CONR⁷R⁸, NR³COR¹⁰ SO₂NR⁴R⁵ und NR³SO₂R⁹ substituiert sind;
R⁷ und R⁸ unabhängig voneinander für Wasserstoff, C₃₋₈-Cycloalkyl oder C₁₋₆-Alkyl stehen, wobei die beiden letztgenannten Gruppen gegebenenfalls durch ein oder mehrere Halogenatome substituiert sind; oder
R⁷ und R⁸ zusammen mit dem Stickstoffatom, an das sie gebunden sind, einen 3- bis 8-gliedrigen gesättigten Ring bilden können, der gegebenenfalls durch Halogen, C₃₋₈-Cycloalkyl oder C₁₋₃-Alkyl substituiert ist;
R⁹ für C₃₋₈-Cycloalkyl oder C₁₋₆-Alkyl (gegebenenfalls substituiert durch Halogenatome) steht;
R¹⁰ für Wasserstoff, Aryl, Heteroaryl, OR₃, C₃₋₈-Cycloalkyl oder C₁₋₆-Alkyl steht, wobei die drei letztgenannten Gruppen gegebenenfalls durch Halogenatome substituiert sind;
R¹¹ und R¹² unabhängig voneinander für Wasserstoff, Aryl, Heteroaryl, C₃₋₈-Cycloalkyl oder C₁₋₈-Alkyl stehen, wobei der Letztere gegebenenfalls durch ein oder mehrere Halogenatome, OR³, =O oder NR¹³R¹⁴ substituiert ist; oder
R¹¹ und R¹² zusammen mit dem Stickstoffatom, an das sie gebunden sind, einen 3- bis 8-gliedrigen Ring bilden können, der gegebenenfalls ein oder mehrere aus O, S und NR³ ausgewählte Atome enthält, wobei dieser Ring selbst gegebenenfalls durch ein oder mehrere C₃₋₈-Cycloalkylreste, C₁₋₃-Alkylreste, Halogene, OR³ oder NR¹³R¹⁴ substituiert ist;
R¹³ und R¹⁴ unabhängig voneinander für Wasserstoff, C₃₋₈-Cycloalkyl oder C₁₋₆-Alkyl stehen, wobei die beiden letztgenannten Gruppen gegebenenfalls durch einen oder mehrere Substituenten unabhängig voneinander ausgewählt aus Halogen, OR³, CN, S O)ₙR⁹ (wobei n = 0, 1 oder 2), CONR⁷R⁸, NR³COR¹⁰, SO₂NR⁴R⁵ und NR³SO₂R⁹ substituiert sind;
oder
R¹³ und R¹⁴ zusammen mit dem Stickstoffatom, an das sie gebunden sind, einen 5- bis 10-gliedrigen ungesättigten Heteroarylring bilden können, der gegebenenfalls ein oder mehrere aus O, N, S(O)ₙ (wobei n = 0, 1 oder 2) und NR³ ausgewählte Atome enthält und selbst gegebenenfalls durch Halogen, CN, S(O)ₙR⁹ (wobei n = 0, 1 oder 2), OR³, C₃₋₈-Cycloalkyl oder C₁₋₃-Alkyl substituiert ist;
R¹⁵ für einen oder mehrere unabhängig voneinander aus Wasserstoff, Halogen, CN, OR³, S(O)ₙR⁹ (wobei n = 0, 1 oder 2), C₃₋₈-Cycloalkyl oder C₁₋₆-Alkyl ausgewählte Substituenten steht, wobei die beiden letztgenannten Gruppen gegebenenfalls durch Halogenatome substituiert sind,
mit der Maßgabe, dass:
• A und D nicht beide für Wasserstoff stehen dürfen.

2. Verbindungen nach Anspruch 1, wobei E für O oder S steht.

3. Verbindungen nach Anspruch 1 oder 2, wobei A und D unabhängig voneinander aus Wasserstoff, Halogen und C₁₋₃-Alkyl ausgewählt sind, wobei der letztgenannte Rest gegebenenfalls durch Halogenatome substituiert ist.

4. Verbindungen nach Anspruch 1 oder 2, wobei A und D unabhängig voneinander aus Wasserstoff, Halogen und CF₃ ausgewählt sind.

5. Verbindungen nach Anspruch 1 oder 2, wobei, wenn A für Wasserstoff steht, D für Halogen oder C₁₋₃-Alkyl steht, wobei der letztgenannte Rest gegebenenfalls durch Halogenatome substituiert ist.

6. Verbindungen nach Anspruch 1 oder 2, wobei, wenn D für Wasserstoff steht, A für Halogen, Phenyl oder C₁₋₃-Alkyl steht, wobei der letztgenannte Rest gegebenenfalls durch Halogenatome substituiert ist.

7. Verbindungen nach Anspruch 1 oder 2, wobei, wenn A für Wasserstoff steht, D für Halogen oder CF₃ steht, und wenn D für Wasserstoff steht, A für Halogen oder CF₃ steht.

8. Verbindung nach einem der Ansprüche 1 bis 7, wobei Y für CR¹R² steht.

9. Verbindung nach Anspruch 8, wobei R¹ und R² beide für Wasserstoff stehen.

10. Verbindung nach einem der Ansprüche 1 bis 9, wobei W für SO₂R⁶ steht.

11. Verbindung nach einem der Ansprüche 1 bis 10, wobei E in der ortho-Stellung bezüglich Y an den aromatischen Ring gebunden ist.

12. Verbindung nach einem der Ansprüche 1 bis 10, wobei E in der meta-Stellung bezüglich Y an den aromatischen Ring gebunden ist.

13. Verbindungen der Formel (I) nach einem der Ansprüche 1 bis 12, ausgewählt aus:
2-[2-[2-Chlor-4-(methylsulfonyl)phenoxy]-4-(trifluormethyl)phenoxy]-*N-*(methylsulfonyl)acetamid;
2-{4-Chlor-3-[2-chlor-4-(methylsulfonyl) phenoxy]phenyl}-*N-*(methylsulfonyl)acetamid;
2-{3-Chlor-5-[2-chlor-4-(methylsulfonyl)phenoxy]phenyl}-*N-*(methylsulfonyl)acetamid;
2-(3-Chlor-5-{[2-fluor-4-(phenylsulfonyl)phenyl]thio}phenyl)-*N-*(methylsulfonyl)acetamid;
2-[3-{2-Chlor-4-[(4-fluorbenzyl)sulfonyl]phenoxy}-5-(trifluormethyl)phenyl]-*N-*(methylsulfonyl)acetamid;
2-[3-[2-Chlor-4-(methylsulfonyl)phenoxy]-5-(trifluormethyl)phenyl]-*N-*(methylsulfonyl)acetamid;
2-[3-[2-Cyano-4-(phenylsulfonyl)phenoxy]-5-(trifluormethyl)phenyl]-*N-*(methylsulfonyl)acetamid;
2-[3-[4-(Ethylsulfonyl)-2-(trifluormethyl) phenoxy]-5-(trifluormethyl)phenyl]-*N-*(methylsulfonyl)acetamid;
2-[3-[2-Cyano-4-(phenylsulfonyl)phenoxy]-5-(trifluormethyl)phenyl]-*N-*(ethylsulfonyl)acetamid;
2-[3-[2-Chlor-4-(phenylsulfonyl)phenoxy]-5-(trifluormethyl)phenyl]-N-(methylsulfonyl)acetamid;
2-[3-[2-Chlor-4-(phenylsulfonyl)phenoxy]-5-(trifluormethyl)phenyl]-N-(ethylsulfonyl)acetamid;
2-[3-[2-Chlor-4-(ethylsulfonyl)phenoxy]-5-(trifluormethyl)phenyl]-N-(ethylsulfonyl)propanamid;
2-[3-{[2-Chlor-4-(ethylsulfonyl)phenyl]thio}-5-(trifluormethyl)phenyl]-N-(ethylsulfonyl)acetamid;
2-[3-{[2-Chlor-4-(ethylsulfonyl)phenyl]thio}-5-(trifluormethyl)phenyl]-N-(methylsulfonyl)acetamid;
N-(tert.-Butylsulfonyl)-2-[3-[2-cyano-4-(phenylsulfonyl)phenoxy] -5-(trifluormethyl)phenyl]acetamid;
2-[3-[2-Cyano-4-(phenylsulfonyl)phenoxy]-5-(trifluormethyl)phenyl]-N-(isopropylsulfonyl)acetamid;
2-[3-[2-Chlor-4-(methylsulfonyl)phenoxy]-5-(trifluormethyl)phenyl]-N-[(2,2,2-trifluorethyl)sulfonyl]acetamid;
2-(4-Chlor-2-{[2-chlor-4-(methylsulfonyl)phenyl]amino}phenoxy)-N-(methylsulfonyl)acetamid;
2-[3-[2-Chlor-4-(methylsulfonyl)phenoxy]-5-(trifluormethyl)phenyl]-N-(ethylsulfonyl)acetamid;
2-[3-[2-Chlor-4-(methylsulfonyl)phenoxy]-5-(trifluormethyl)phenyl]-N-(propylsulfonyl)acetamid;
N-(Benzylsulfonyl)-2-[3-[2-chlor-4-(methylsulfonyl)phenoxy]-5-(trifluormethyl)phenyl]acetamid;
N-(Ethylsulfonyl)-2-[3-[4-(methylsulfonyl)-2-(trifluormethyl)phenoxy]-5-(trifluormethyl)phenyl]acetamid;
2-[3-[4-(Methylsulfonyl)-2-(trifluormethyl)phenoxy]-5-(trifluormethyl)phenyl]-N-(propylsulfonyl)acetamid;
2-{5-[2-Chlor-4-(ethylsulfonyl)phenoxy]biphenyl-3-yl}-N-(ethylsulfonyl)acetamid;
2-{3-Chlor-5-[4-(methylsulfonyl)-2-(trifluormethyl)phenoxy]phenyl}-*N-*[(trifluormethyl)sulfonyl]acetamid;
2-{3-Chlor-5-[2-chlor-4-(4-fluorbenzoyl)phenoxy]phenyl}-N-[(2,2,2-trifluorethyl)sulfonyl]acetamid;
2-[3-[2-Cyano-4-(phenylsulfonyl)phenoxy]-5-(trifluormethyl)phenyl]-N-(propylsulfonyl)acetamid;
2-{3-[2-Chlor-4-(ethylsulfonyl)phenoxy]-5-fluorphenyl}-N-(propylsulfonyl)acetamid;
2-{3-Chlor-5-[2-chlor-4-(ethylsulfonyl)phenoxy]phenyl}-N-(propylsulfonyl)acetamid;
2-{3-Chlor-5-[2-chlor-4-(ethylsulfonyl)phenoxy]phenyl}-N-(ethylsulfonyl)acetamid;
2-{3-Chlor-5-[2-chlor-4-(ethylsulfonyl)phenoxy]phenyl}-*N-*(isobutylsulfonyl)acetamid;
und deren pharmazeutisch annehmbare Salze.

14. Pharmazeutische Zusammensetzung, enthaltend eine Verbindung der Formel (I) oder ein pharmazeutisch annehmbares Salz davon nach einem der Ansprüche 1 bis 13 zusammen mit einem pharmazeutisch annehmbaren Adjuvans, Verdünnungsmittel oder Träger.

15. Verbindungen der Formel (I) und deren pharmazeutisch annehmbare Salze nach einem der Ansprüche 1 bis 13 zur Verwendung als Medikament.

16. Verbindungen der Formel (I) und deren pharmazeutisch annehmbare Salze nach einem der Ansprüche 1 bis 13 zur Verwendung bei der Behandlung oder Prophylaxe von Asthma oder COPD.

## Revendications

1. Composé de formule (I) ou un sel pharmaceutiquement acceptable de celui-ci : 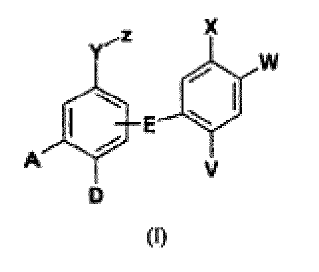 dans laquelle
A et D sont choisis indépendamment parmi hydrogène, halogène, phényle ou alkyle en C₁₋₃, ce dernier étant éventuellement substitué par des atomes d'halogène ;
E est O, S, NR^{3'} ou CH₂ ;
V est halogène, CN ou alkyle en C₁₋₃, ce dernier étant éventuellement substitué par des atomes d'halogène ;
W est choisi parmi SO₂R⁶ et COR⁴ ;
X est hydrogène ou CF₃ ;
Y est choisi parmi O-CR¹R², CR¹R² et CR¹R²CR¹R² ;
Z est CONHSO₂R⁶ ;
R¹ et R² indépendamment représentent un atome d'hydrogène, halogène, cycloalkyle en C₃₋₆ ou un groupement alkyle en C₁₋₆, les deux derniers groupements étant éventuellement substitués par un ou plusieurs atomes d'halogène ; ou
R¹ et R² ensemble peuvent former un cycle à 3-8 chaînons contenant éventuellement un ou plusieurs atomes choisis parmi O, S, NR³ et lui-même éventuellement substitué par un ou plusieurs halogènes, cycloalkyles en C₃₋₈ ou alkyles en C₁₋₃, ces deux derniers groupements étant éventuellement substitués par un ou plusieurs halogènes ou NR⁴R⁵ ;
R³ représente hydrogène, cycloalkyle en C₃₋₈ ou alkyle en C₁₋₆, ces deux derniers groupements étant éventuellement substitués par des atomes d'halogène ;
R^{3'} représente hydrogène, cycloalkyle en C₃₋₈ ou alkyle en C₁₋₆, ces deux derniers groupements étant éventuellement substitués par des atomes d'halogène ;
R⁴ et R⁵ indépendamment représentent hydrogène, cycloalkyle en C₃₋₈ ou alkyle en C₁₋₆, ces deux derniers groupements étant éventuellement substitués par un ou plusieurs substituants choisis indépendamment parmi halogène, CN, cycloalkyle en C₃₋₇, alkyle en C₁₋₆, OR³ et NR⁷R⁸, aryle, hétéroaryle, S(O)ₙR⁹ (où n = 0, 1 ou 2), CONR⁷R⁸, NR³COR¹⁰, SO₂NR⁴R⁵ et NR³SO₂R⁹ ; ou
R⁴ et R⁵ ensemble avec l'atome d'azote auquel ils sont liés peuvent former un cycle saturé à 3-8 chaînons contenant éventuellement un ou plusieurs atomes choisis parmi O, S, S(O)ₙ (où n = 0, 1 ou 2), NR³, et lui-même éventuellement substitué par un ou plusieurs halogènes, OR³, cycloalkyles en C₃₋₈ ou alkyles en C₁₋₆, ces deux derniers groupements étant éventuellement substitués par un ou plusieurs halogènes ;
R⁶ représente aryle, hétéroaryle, cycloalkyle en C₃₋₈ ou alkyle en C₁₋₆, tous étant éventuellement substitués par un ou plusieurs substituants choisis indépendamment parmi halogène, cycloalkyle en C₃₋₇, alkyle en C₁₋₆, OR³, CN, NR⁷R⁸, aryle, hétéroaryle, S(O)ₙR⁹ (où n = 0, 1 ou 2), CONR⁷R⁸, NR³COR¹⁰, SO₂NR⁴R⁵ et NR³SO₂R⁹ ;
R⁷ et R⁸ indépendamment représentent hydrogène, cycloalkyle en C₃₋₈ ou alkyle en C₁₋₆, ces deux derniers groupements étant éventuellement substitués par un ou plusieurs atomes d'halogène ;
ou
R⁷ et R⁸ ensemble avec l'atome d'azote auquel ils sont liés peuvent former un cycle saturé à 3-8 chaînons, éventuellement substitué par halogène, cycloalkyle en C₃₋₈ ou alkyle en C₁₋₃ ;
R⁹ représente cycloalkyle en C₃₋₈ ou alkyle en C₁₋₆ (éventuellement substitué par des atomes d'halogène) ;
R¹⁰ représente hydrogène, aryle, hétéroaryle, OR₃, cycloalkyle en C₃₋₈ ou alkyle en C₁₋₆, ces trois derniers groupements étant éventuellement substitués par des atomes d'halogène ;
R¹¹ et R¹² indépendamment représentent hydrogène, aryle, hétéroaryle, cycloalkyle en C₃₋₈ ou alkyle en C₁₋₈, ce dernier étant éventuellement substitué par un ou plusieurs atomes d'halogène, OR³, =O ou NR¹³R¹⁴ ; ou
R¹¹ et R¹² ensemble avec l'atome d'azote auquel ils sont liés peuvent former un cycle à 3-8 chaînons contenant éventuellement un ou plusieurs atomes choisis parmi O, S, NR³, ce cycle est lui-même éventuellement substitué par un ou plusieurs cycloalkyles en C₃₋₈, alkyles en C₁₋₃, halogènes, OR³ ou NR¹³R¹⁴ _{;}
R¹³ et R¹⁴ indépendamment représentent hydrogène, cycloalkyle en C₃₋₈ ou alkyle en C₁₋₆, ces deux derniers groupements étant éventuellement substitués par un ou plusieurs substituants choisis indépendamment parmi halogène, OR³, CN, S(O)ₙR⁹ (où n = 0, 1 ou 2), CONR⁷R⁸ , NR³COR¹⁰, SO₂NR⁴R⁵ et NR³SO₂R⁹ ;
ou
R¹³ et R¹⁴ ensemble avec l'atome d'azote auquel ils sont liés peuvent former un cycle hétéroaryle insaturé à 5-10 chaînons contenant éventuellement un ou plusieurs atomes choisis parmi O, N, S(O)ₙ (où n = 0, 1 ou 2), NR³, et lui-même éventuellement substitué par halogène, CN, S(O)ₙR⁹ (où n = 0, 1 ou 2), OR³, cycloalkyle en C₃₋₈ ou alkyle en C₁₋₃ ;
R¹⁵ représente un ou plusieurs substituants choisis indépendamment parmi hydrogène, halogène, CN, OR³, S(O)ₙR⁹ (où n = 0, 1 ou 2), cycloalkyle en C₃₋₈ ou alkyle en C₁₋₆, ces deux derniers groupements étant éventuellement substitués par des atomes d'halogène,
à condition que :
• A et D ne puissent pas tous deux être hydrogène.

2. Composé selon la revendication 1, **caractérisé en ce que** E est O ou S.

3. Composé selon la revendication 1 ou 2, **caractérisé en ce que** A et D sont choisis indépendamment parmi hydrogène, halogène ou alkyle en C₁₋₃, ce dernier étant éventuellement substitué par des atomes d'halogène.

4. Composé selon la revendication 1 ou 2, **caractérisé en ce que** A et D sont choisis indépendamment parmi hydrogène, halogène ou CF₃.

5. Composé selon la revendication 1 ou 2, **caractérisé en ce que** lorsque A est hydrogène, D est halogène ou alkyle en C₁₋₃, ce dernier étant éventuellement substitué par des atomes d'halogène.

6. Composé selon la revendication 1 ou 2, **caractérisé en ce que** D est hydrogène, A est halogène, phényle ou alkyle en C₁₋₃, ce dernier étant éventuellement substitué par des atomes d'halogène.

7. Composé selon la revendication 1 ou 2, **caractérisé en ce que** lorsque A est hydrogène, D est halogène ou CF₃ ; lorsque D est hydrogène, A est halogène ou CF₃.

8. Composé selon l'une quelconque des revendications 1 à 7, **caractérisé en ce qu'**Y est CR¹R².

9. Composé selon la revendication 8, **caractérisé en ce que** R¹ et R² sont tous deux hydrogène.

10. Composé selon l'une quelconque des revendications 1 à 9, **caractérisé en ce que** W est SO₂R⁶.

11. Composé selon l'une quelconque des revendications 1 à 10, **caractérisé en ce que** E est relié au cycle aromatique en position *ortho* par rapport à Y.

12. Composé selon l'une quelconque des revendications 1 à 10, **caractérisé en ce que** E est relié au cycle aromatique en position méta par rapport à Y.

13. Composé de formule (I) tel que défini dans l'une quelconque des revendications 1 à 12, choisi parmi :
le 2-[2-[2-chloro-4-(méthylsulfonyl)phénoxy]-4-(trifluorométhyl)phénoxy]-*N-*(méthylsulfonyl)acétamide ;
le 2-{4-chloro-3-[2-chloro-4-(méthylsulfonyl)phénoxy]phényl}-*N-*(méthylsulfonyl)acétamide ;
le 2-{3-chloro-5-[2-chloro-4-(méthylsulfonyl)phénoxy]phényl}-*N-*(méthylsulfonyl)acétamide ;
le 2-(3-chloro-5-{[2-fluoro-4-(phénylsulfonyl)phényl]thio}phényl)-N-(méthylsulfonyl)acétamide ;
le 2-[3-{2-chloro-4-[(4-fluorobenzyl)sulfonyl]phénoxy}-5-(trifluorométhyl)phényl]-*N-*(méthylsulfonyl)acétamide ;
le 2-[3-[2-chloro-4-(méthylsulfonyl)phénoxy]-5-(trifluorométhyl)phényl]-*N-*(méthylsulfonyl)acétamide ;
le 2-[3-[2-cyano-4-(phénylsulfonyl)phénoxy]-5-(trifluorométhyl)phényl]-*N-*(méthylsulfonyl)acétamide ;
le 2-[3-[4-(éthylsulfonyl)-2-(trifluorométhyl)phénoxy]-5-(trifluorométhyl)phényl]-*N-*(méthylsulfonyl)acétamide ;
le 2-[3-[2-cyano-4-(phénylsulfonyl)phénoxy]-5-(trifluorométhyl)phényl]-*N-*(éthylsulfonyl)acétamide ;
le 2-[3-[2-chloro-4-(phénylsulfonyl)phénoxy]-5-(trifluorométhyl)phényl]-*N-*(méthylsulfonyl)acétamide ;
le 2-[3-[2-chloro-4-(phénylsulfonyl)phénoxy]-5-(trifluorométhyl)phényl]-*N-*(éthylsulfonyl)acétamide ;
le 2-[3-[2-chloro-4-(éthylsulfonyl)phénoxy]-5-(trifluorométhyl)phényl]-*N-*(éthylsulfonyl)propanamide ;
le 2-[3-{[2-chloro-4-(éthylsulfonyl)phényl]thio}-5-(trifluorométhyl)phényl]-*N-*(éthylsulfonyl)acétamide ;
le 2-[3-{[2-chloro-4-(éthylsulfonyl)phényl]thio}-5-(trifluorométhyl)phényl]-*N-*(méthylsulfonyl)acétamide ;
le N-(tert-butylsulfonyl)-2-[3-[2-cyano-4-(phénylsulfonyl)phénoxy]-5-(trifluorométhyl)phényl]acétamide ;
le 2-[3-[2-cyano-4-(phénylsulfonyl)phénoxy]-5-(trifluorométhyl)phényl]-*N-*(isopropylsulfonyl)acétamide ;
le 2-[3-[2-chloro-4-(méthylsulfonyl)phénoxy]-5-(trifluorométhyl)phényl]*-N*-[(2,2,2-trifluoroéthyl)sulfonyl]acétamide ;
le 2-(4-chloro-2-{[2-chloro-4-(méthylsulfonyl)phényl]amino}phénoxy-*N-*(méthylsulfonyl)acétamide ;
le 2-[3-[2-chloro-4-(méthylsulfonyl)phénoxy]-5-(trifluorométhyl)phényl]-*N-*(éthylsulfonyl)acétamide ;
le 2-[3-[2-chloro-4-(méthylsulfonyl)phénoxy]-5-(trifluorométhyl)phényl]-*N-*(propylsulfonyl)acétamide ;
le N-(benzylsulfonyl)-2-[3-[2-chloro-4-(méthylsulfonyl)phénoxy]-5-(trifluorométhyl)phényl]acétamide ;
le N-(éthylsulfonyl)-2-[3-[4-(méthylsulfonyl)-2-(trifluorométhyl)phénoxy]-5-(trifluorométhyl)phényl]acétamide ;
le 2-[3-[4-(méthylsulfonyl)-2-(trifluorométhyl)phénoxy]-5-(trifluorométhyl)phényl]-*N-*(propylsulfonyl)acétamide ;
le 2-{5-[2-chloro-4-(éthylsulfonyl)phénoxy]biphényl-3-yl}-*N-*(éthylsulfonyl)acétamide ;
le 2-{3-chloro-5-[4-(méthylsulfonyl)-2-(trifluorométhyl)phénoxy]phényl}-*N-*[(trifluorométhyl)sulfonyl]acétamide ;
le 2-{3-chloro-5-[2-chloro-4-(4-fluorobenzoyl)phénoxy]phényl}-*N-*[(2,2,2-trifluoroéthyl)sulfonyl]acétamide ;
le 2-[3-[2-cyano-4-(phénylsulfonyl)phénoxy]-5-(trifluorométhyl)phényl]-*N-*(propylsulfonyl)acétamide ;
le 2-{3-[2-chloro-4-(éthylsulfonyl)phénoxy]-5-fluorophényl}-*N-*(propylsulfonyl)acétamide ;
le 2-{3-chloro-5-[2-chloro-4-(éthylsulfonyl)phénoxy]phényl}-*N-*(propylsulfonyl)acétamide ;
le 2-{3-chloro-5-[2-chloro-4-(éthylsulfonyl)phénoxy]phényl}-*N-*(éthylsulfonyl)acétamide ;
le 2-{3-chloro-5-[2-chloro-4-(éthylsulfonyl)phénoxy]phényl}-*N-*(isobutylsulfonyl)acétamide ;
et les sels pharmaceutiquement acceptables de ceux-ci.

14. Composition pharmaceutique comprenant un composé de formule (I) ou un sel pharmaceutiquement acceptable de celui-ci, tel que défini dans l'une quelconque des revendications 1 à 13, en association avec un adjuvant, un diluant ou un support pharmaceutiquement acceptables.

15. Composé de formule (I) ou un sel pharmaceutiquement acceptable de celui-ci, tel que défini dans l'une quelconque des revendications 1 à 13, destiné à être utilisé comme médicament.

16. Composé de formule (I) ou un sel pharmaceutiquement acceptable de celui-ci, tel que défini dans l'une quelconque des revendications 1 à 13, destiné à être utilisé dans le traitement ou la prophylaxie de l'asthme ou de la BPCO.
